# EUROPEAN PATENT APPLICATION

(11) **EP 1 736 162 A1**
(43) Date of publication of application: **27.12.2006**
(21) Application number: 05727648.7
(22) Date of filing: 30.03.2005
(51) Int. Cl.: A61K 35/12, A61K 39/395, A61K 48/00, A61P 25/00, C07K 16/46, C12N 5/10, C12N 15/09

(54) **REMEDY FOR PRION DISEASE AND METHOD OF PRODUCING THE SAME**

(30) Priority: 30.03.2004 JP 2004100649
(71) Applicant: Renomedix Institute Inc., Ishikari-shi, Hokkai-do 0613241 (JP)
(72) Inventor: FUJINAGA, Kei, Hokkaido 0640941 (JP); SHINAGAWA, Morikazu, Tsukuba-shi, Ibaraki 3050035 (JP); NIITSU, Yoshiro, Sapporo-shi, Hokkaido 0640823 (JP); HAMADA, Hirofumi, Hokkaido 0640959 (JP); HORIUCHI, Motohiro, Hokkaido 0028005 (JP); HONMOU, Osamu, Sapporo-shi Hokkaido 0640801 (JP); UMETANI, Atsushi, 1830045 (JP)
(74) Representative: Forstmeyer, Dietmar
(86) International application number: PCT/JP2005/006189
(87) International publication number: WO 2005/094846

(57) **Abstract**

It is intended to provide a drug which is efficacious in treating a prion disease and has a high safety. A remedy for a prion disease which contains a mesenchymal stem cell as the active ingredient and a method of producing the same. A remedy for a prion disease which contains a mesenchymal stem cell, in particular, a mesenchymal stem cell having an anti-prion antibody gene transferred thereinto as the active ingredient and a method of producing the same. These remedies can not only prevent the progress of a prion disease but also contribute to the recovery of nerve dysfunction caused by the disease.

SEQUENCE LISTING

<110> RenoMedix Inst. Inc.

<120> Agent for treating prion disease and production method thereof

<130> PCT2185RM

<150> JP2004-100649
<151> 2004-03-30

<160> 41

<170> PatentIn version 3.1

<210> 1
<211> 1509
<212> DNA
<213> Artificial Sequence

<220>
<223> Anti-PrP mAb H chain

<400> 1
gaattcgccc ttctaatacg actcactata gggcaagcag tggtatcaac gcagagtacg 60

cgggaacata tgtccaatgt cctctccaca ggcactgaac acactgactc taaccatggg 120

atggagctgg atctttctcc tcttcctgtc aggaactgca ggtgtcctct ctgaggtcca 180

gctgcaacag tctggacctg aggtggtgaa gcctggggct tcattgaaga taccctgcaa 240

ggcttctgga tacacattca ctgactacaa catggactgg gtgaagcaga gccatggaaa 300

gagccttgag tggattggag atattaatcc taacaatggt ggtactatct acaaccacaa 360

cttcacggac aaggccacat tgactgtaga caagtcctcc agcacagcct acatggagct 420

ccgcagcctg acatctgagg acactgcagt ctattactgt gcaagggcta cttcgttagt 480

agactttgac tactggggcc aaggcaccac tctcacagtc tcctcagcca aaacgacacc 540

cccatctgtc tatccactgg cccctggatc tgctgcccaa actaactcca tggtgaccct 600

gggatgcctg gtcaagggct atttccctga gccagtgaca gtgacctgga actctggatc 660

cctgtccagc ggtgtgcaca ccttcccagc tgtcctgcag tctgacctct acactctgag 720

cagctcagtg actgtcccct ccagcacctg gcccagccag accgtcacct gcaacgttgc 780

ccacccggcc agcagcacca aggtggacaa gaaaattgtg cccagggatt gtggttgtaa 840

gccttgcata tgtacagtcc cagaagtatc atctgtcttc atcttccccc caaagcccaa 900

ggatgtgctc accattactc tgactcctaa ggtcacgtgt gttgtggtag acatcagcaa 960

ggatgatccc gaggtccagt tcagctggtt tgtagatgat gtggaggtgc acacagctca 1020

gacgcaaccc cgggaggagc agatcaacag cactttccgt tcagtcagtg aacttcccat 1080

catgcaccag gactggctca atggcaagga gttcaaatgc agggtcaaca gtgcagcttt 1140

ccctgccccc atcgagaaaa ccatctccaa aaccaaaggc agaccgaagg ctccacaggt 1200

gtacaccatt ccacctccca aggagcagat ggccaaggat aaagtcagtc tgacctgcat 1260

gataacaaac ttcttccctg aagacattac tgtggagtgg cagtggaatg ggcagccagc 1320

ggagaactac aagaacactc agcccatcat ggacacagat ggctcttact tcgtctacag 1380

caagctcaat gtgcagaaga gcaactggga ggcaggaaat actttcacct gctctgtgtt 1440

acatgagggc ctgcacaacc accatactga gaagagcctc tcccactctc ctggtaaatg 1500

agatctcgg 1509

<210> 2
<211> 836
<212> DNA
<213> Artificial Sequence

<220>
<223> Anti-PrP mAb L chain

<400> 2
gaattcgccc ttctaatacg actcactata gggcaagcag tggtatcaac gcagagtacg 60

cggggactga tcagtctcct caggctgtct cctcaggttg cctcctcaaa atgaagttgc 120

ctgttaggct gttggtgctg atgttctgga ttcctgcttc cagcagtgat gttttgatga 180

cccaaactcc actctccctg cctgtcagtc ttggagatca agcctccatc tcttgcagat 240

ctagtcagag cattgtacat actaatggaa acacctattt agaatggttc ctgcagaaac 300

caggccagtc tcccaagctc ctgatctaca aagtttccaa ccgattttct ggggtcccag 360

acaggttcag tggcagtgga tcagggacag atttcacact caagatcagc agagtggagg 420

ctgaggatct gggagtttat tactgctttc aaggttcact tgttccgtac acgttcggag 480

gggggaccaa gctggaaata aaacgggctg atgctgcacc aactgtatcc atcttcccac 540

cctcgagtga gcagttaaca tctggaggtg cctcagtcgt gtgcttcttg aacaacttct 600

accccaaaga catcaatgtc aagtggaaga ttgatggcag tgaacgacaa aatggcgtcc 660

tgaacagttg gactgatcag gacagcaaag acagcaccta cagcatgagc agcaccctca 720

cgttgaccaa ggacgagtat gaacgacata acagctatac ctgtgaggcc actcacaaga 780

catcaacttc acccattgtc aagagcttca acaggaatga gtgttaagga tccgcg 836

<210> 3
<211> 1458
<212> DNA
<213> Artificial Sequence

<220>
<223> Anti-PrP mAb H chain

<400> 3
gaattcgccc ttctaatacg actcactata gggcaagcag tggtatcaac gcagagtacg 60

cgggactcta accatggaat ggatctggat ctttctcttc atcctgtcag gaactgcagg 120

tgtccaatcc caggttcagc tgctgcagtc tggagctgaa ctggcgaggc ctggggcttc 180

agtgaagctg tcctgcaagg gttctggcta caccttcaca agctatagta taagttgggt 240

gaagcagaga actggacagg gccttgagtg gattggagag atttatccta gaagtggtaa 300

tacttactac aatgagaagt tcaaggacaa ggccacactg actgcagaca aatcctccag 360

cacagcgtac atggagctcc gcagcctgac atctgaggac tctgcggtct atttctgtgc 420

aacggattac ctgtttgctt actggggcca agggacgctg gtcactgtct ctgcagccaa 480

aacgacaccc ccatctgtct atccactggc ccctggatct gctgcccaaa ctaactccat 540

ggtgaccctg ggatgcctgg tcaagggcta tttccctgag ccagtgacag tgacctggaa 600

ctctggatcc ctgtccagcg gtgtgcacac cttcccagct gtcctgcagt ctgacctcta 660

cactctgagc agctcagtga ctgtcccctc cagcacctgg cccagccaga ccgtcacctg 720

caacgttgcc cacccggcca gcagcaccaa ggtggacaag aaaattgtgc ccagggattg 780

tggttgtaag ccttgcatat gtacagtccc agaagtatca tctgtcttca tcttcccccc 840

aaagcccaag gatgtgctca ccattactct gactcctaag gtcacgtgtg ttgtggtaga 900

catcagcaag gatgatcccg aggtccagtt cagctggttt gtagatgatg tggaggtgca 960

cacagctcag acgcaacccc gggaggagca gatcaacagc actttccgtt cagtcagtga 1020

acttcccatc atgcaccagg actggctcaa tggcaaggag ttcaaatgca gggtcaacag 1080

tgcagctttc cctgccccca tcgagaaaac catctccaaa accaaaggca gaccgaaggc 1140

tccacaggtg tacaccattc cacctcccaa ggagcagatg gccaaggata aagtcagtct 1200

gacctgcatg ataacaaact tcttccctga agacattact gtggagtggc agtggaatgg 1260

gcagccagcg gagaactaca agaacactca gcccatcatg gacacagatg gctcttactt 1320

cgtctacagc aagctcaatg tgcagaagag caactgggag gcaggaaata ctttcacctg 1380

ctctgtgtta catgagggcc tgcacaacca ccatactgag aagagcctct cccactctcc 1440

tggtaaatga gatctcgg 1458

<210> 4
<211> 757
<212> DNA
<213> Artificial Sequence

<220>
<223> Anti-PrP mAb L chain

<400> 4
gaattcgccc ttattgtcat tgcagtcagg actcagcatg gacatgaggg ctcctgcaca 60

gatttttggc ttcttgttgc tcttgtttcc aggtaccaga tgtgacatcc agatgaccca 120

gtctccatcc tccttatctg cctctctggg agaaagagtc agtctcactt gtcgggcaag 180

tcaggacatt ggtagtagtt taaactggct tcaacaggaa ccagatggaa ctattaaacg 240

cctgatctac gccacatcca gtttagattc tggtgtcccc aaaaggttca gtggcagtag 300

gtctgggtca gattattctc tcaccatcag cagccttgag tctgaagatt ttgtagacta 360

ttactgtctg caatatgcaa aatctccgta cacgttcgga ggggggacca agctggaaat 420

aaaacgggct gatgctgcac caactgtatc catcttccca ccctcgagtg agcagttaac 480

atctggaggt gcctcagtcg tgtgcttctt gaacaacttc taccccaaag acatcaatgt 540

caagtggaag attgatggca gtgaacgaca aaatggcgtc ctgaacagtt ggactgatca 600

ggacagcaaa gacagcacct acagcatgag cagcaccctc acgttgacca aggacgagta 660

tgaacgacat aacagctata cctgtgaggc cactcacaag acatcaactt cacccattgt 720

caagagcttc aacaggaatg agtgttaagg atccgcg 757

<210> 5
<211> 1560
<212> DNA
<213> Artificial Sequence

<220>
<223> Anti-PrP mAb H chain

<400> 5
gaattcgccc ttctaatacg actcactata gggcaagcag tggtatcaac gcagagtacg 60

cggggagctc tgacagagga ggccagtcct ggaattgatt cctagttcct cacgttcagt 120

gatgagtatt gatcacagac ccctcaccat gaacttcggg ctcagattga ttttccttgt 180

cctcacttta aaaggtgtcc agtgtgacgt gaagctggtg gagtctgggg aaggcttagt 240

gaagcctgga gggtccctga aactctcctg tgcagcctct ggaatcactt tcagtaggta 300

tgccatgtct tgggttcgcc agactccaga gaagaggctg gagtgggtcg catacattag 360

tagtggtggt gattacatca actatgcaga cactgtgaag ggccgattca ccatctccag 420

agacaatgcc aggaacaccc tgtacctgca aatgagcagt ctgaagtctg aggacacagc 480

catgtattac tgtacaagag taactccata ttggtacttc gatgtctggg gcacagggac 540

cacggtcacc gtctcctcag ccaaaacaac agccccatcg gtctatccac tggcccctgt 600

gtgtggaggt acaactggct cctcggtgac tctaggatgc ctggtcaagg gttatttccc 660

tgagccagtg accttgacct ggaactctgg atccctgtcc agtggtgtgc acaccttccc 720

agctctcctg cagtctgacc tctacaccct cagcagctca gtgactgtaa cctcgagcac 780

ctggcccagc cagtccatca cctgcaatgt ggcccacccg gcaagcagca ccaaggtgga 840

caagaaaatt gagcccagag ggcccacaat caagccctgt cctccatgca aatgcccagc 900

acctaacctc ttgggtggac catccgtctt catcttccct ccaaagatca aggatgtact 960

catgatctcc ctgagcccca tagtcacatg tgtggtggtg gatgtgagcg aggatgaccc 1020

agatgtccag atcagctggt ttgtgaacaa cgtggaagta cacacagctc agacacaaac 1080

ccatagagag gattacaaca gtactctccg ggtggtcagt gccctcccca tccagcacca 1140

ggactggatg agtggcaagg agttcaaatg caaggtcaac aacaaagacc tcccagcgcc 1200

catcgagaga accatctcaa aacccaaagg gtcagtaaga gctccacagg tatatgtctt 1260

gcctccacca gaagaagaga tgactaagaa acaggtcact ctgacctgca tggtcacaga 1320

cttcatgcct gaagacattt acgtggagtg gaccaacaac gggaaaacag agctaaacta 1380

caagaacact gaaccagtcc tggactctga tggttcttac ttcatgtaca gcaagctgag 1440

agtggaaaag aagaactggg tggaaagaaa tagctactcc tgttcagtgg tccacgaggg 1500

tctgcacaat caccacacga ctaagagctt ctcccggact ccgggtaaat gagatctcgg 1560

<210> 6
<211> 815
<212> DNA
<213> Artificial Sequence

<220>
<223> Anti-PrP mAb L chain

<400> 6
gaattcgccc ttaagcagtg gtatcaacgc agagtacgcg gggagctgcc aggagcctaa 60

taaagcatcc tctcttccag ctctcagaga tggagacaga cacactcctg ctatgggtgc 120

tgctgctctg ggttccaggt tccacaggtg acattgtgct gacccaatct ccagcttctt 180

tgggtgtgtc tctagggcag agggccacca tatcctgcag agccagtgaa agtgttgata 240

gttatggcaa tagttttatg cactggtacc agcagaaacc aggacagcca cccaaagtcc 300

tcatctatcg tgcatccaat cgagaatctg ggatccctgc caggttcagt ggcagtgggt 360

ctaggacaga cttcaccctc accattaatc ctgtggaggc tgatgatgtt gcaacctatt 420

actgtcagca aagtaatgag gatccgtata cattcggagg ggggaccaag ctggaaataa 480

aacgggctga tgctgcacca actgtatcca tcttcccacc ctcgagtgag cagttaacat 540

ctggaggtgc ctcagtcgtg tgcttcttga acaacttcta ccccaaagac atcaatgtca 600

agtggaagat tgatggcagt gaacgacaaa atggcgtcct gaacagttgg actgatcagg 660

acagcaaaga cagcacctac agcatgagca gcaccctcac gttgaccaag gacgagtatg 720

aacgacataa cagctatacc tgtgaggcca ctcacaagac atcaacttca cccattgtca 780

agagcttcaa caggaatgag tgttaaggat ccgcg 815

<210> 7
<211> 30
<212> DNA
<213> Artificial Sequence

<220>
<223> Adapter sequence

<400> 7
aagcagtggt atcaacgcag agtacgcggg 30

<210> 8

<211> 45
<212> DNA
<213> Artificial Sequence

<220>
<223> Universal primer

<400> 8
ctaatacgac tcactatagg gcaagcagtg gtatcaacgc agagt 45


<210> 9
<211> 36
<212> DNA
<213> Artificial Sequence

<220>
<223> 5' RACE primer

<400> 9
tcactcgagg gtgggaagat ggatacagtt ggtgca 36


<210> 10
<211> 32
<212> DNA
<213> Artificial Sequence

<220>
<223> PCR primer

<400> 10
accctcgagt gagcagttaa catctggagg tg 32


<210> 11
<211> 36
<212> DNA
<213> Artificial Sequence

<220>
<223> PCR primer

<400> 11
cgcggatcct taacactcat tcctgttgaa gctctt 36


<210> 12
<211> 23
<212> DNA
<213> Artificial Sequence

<220>
<223> 5' RACE primer

<400> 12
cttgaccagg catcccaggg tca 23


<210> 13
<211> 25
<212> DNA
<213> Artificial Sequence

<220>
<223> PCR primer

<400> 13
cctggatctg ctgcccaaac taact 25


<210> 14
<211> 25
<212> DNA
<213> Artificial Sequence

<220>
<223> PCR primer

<400> 14
cggaaagtgc tgttgaactg ctcct 25

<210>  15
<211>  24 .
<212>  DNA
<213>  Artificial Sequence

<220>
<223>  PCR primer

<400>  15
aggtgcacac agctcagacg caac                                              24


<210>  16
<211>  33
<212>  DNA
<213>  Artificial Sequence

<220>
<223>  PCR primer

<400>  16
ccgagatctc atttaccagg agagtgggag agg                                    33


<210>  17
<211>  25
<212>  DNA
<213>  Artificial Sequence

<220>
<223>  PCR primer

<400>  17
cggagatttt gcatattgca gcagt                                             25


<210>  18
<211>  28
<212>  DNA
<213>  Artificial Sequence

<220>
<223>  PCR primer

<400>  18
aatagtctac aaaatcttca gactcaag                                          28


<210>  19
<211>  23
<212>  DNA
<213>  Artificial Sequence

<220>
<223>  PCR primer

<400>  19
cagtctcact tgtcgggcaa gtc                                               23


<210>  20
<211>  26
<212>  DNA
<213>  Artificial Sequence

<220>
<223>  PCR primer

<400>  20
atctaaactg gatgtggcgt agatca                                            26


<210>  21
<211>  23
<212>  DNA
<213>  Artificial Sequence

<220>
<223>  PCR primer

<400> 21
gagccagtga ccttgacctg gaa 23

<210> 22
<211> 23
<212> DNA
<213> Artificial Sequence

<220>
<223> 5' RACE primer

<400> 22
cacattgcag gtgatggact ggc 23

<210> 23
<211> 23
<212> DNA
<213> Artificial Sequence

<220>
<223> PCR primer

<400> 23
agtacacagc tcagacacaa acc 23

<210> 24
<211> 23
<212> DNA
<213> Artificial Sequence

<220>
<223> PCR primer

<400> 24
ctcatccagt cctggtgctg gat 23

<210> 25
<211> 24
<212> DNA
<213> Artificial Sequence

<220>
<223> PCR primer

<400> 25
ccgagatctc atttacccgg agtc 24

<210> 26
<211> 26
<212> DNA
<213> Artificial Sequence

<220>
<223> PCR primer

<400> 26
attgtcattg cagtcaggac tcagca 26

<210> 27
<211> 24
<212> DNA
<213> Artificial Sequence

<220>
<223> PCR primer

<400> 27
actcagcatg gacatgaggg ctcc 24

<210> 28
<211> 84
<212> DNA
<213> Artificial Sequence

<220>
<223>  Forward primer for RT-PCR

<400>  28
aagcaagagc attttcctga tcaggagccc aaatcttctg acaaaactca cacatcccca      60

ccgtccccag cacctgaact cctg                                             84


<210>  29
<211>  41
<212>  DNA
<213>  Artificial Sequence

<220>
<223>  Reverse primer for RT-PCR

<400>  29
cttcgaccag tctagaagca tcctcgtgcg accgcgagag c                          41


<210>  30
<211>  1443
<212>  DNA
<213>  Artificial Sequence

<220>
<223>  Chimeric anti-PrP mAb H chain

<400>  30
atgtcctctc cacaggcact gaacacactg actctaacca tgggatggag ctggatcttt      60

ctcctcttcc tgtcaggaac tgcaggtgtc ctctctgagg tccagctgca acagtctgga     120

cctgaggtgg tgaagcctgg ggcttcattg aagataccct gcaaggcttc tggatacaca     180

ttcactgact acaacatgga ctgggtgaag cagagccatg gaaagagcct tgagtggatt     240

ggagatatta atcctaacaa tggtggtact atctacaacc acaacttcac ggacaaggcc     300

acattgactg tagacaagtc ctccagcaca gcctacatgg agctccgcag cctgacatct     360

gaggacactg cagtctatta ctgtgcaagg gctacttcgt tagtagactt tgactactgg     420

ggccaaggca ccactctcac agtctcctca gcgtcgacca agggcccatc ggtcttcccc     480

ctggcaccct cctccaagag cacctctggg ggcacagcgg ccctgggctg cctggtcaag     540

gactacttcc ccgaaccggt gacggtgtcg tggaactcag gcgccctgac cagcggcgtg     600

cacaccttcc cggctgtcct acagtcctca ggactctact ccctcagcag cgtggtgacc     660

gtgccctcca gcagcttggg cacccagacc tacatctgca acgtgaatca caagcccagc     720

aacaccaagg tggacaagaa agttgagccc aaatcttgtg acagaactca cacatgccca     780

ccgtgcccag cacctgaact cctgggggga ccgtcagtct tcctcttccc cccaaaaccc     840

aaggacaccc tcatgatctc ccggacccct gaggtcacat gcgtggtggt ggacgtgagc     900

cacgaagacc ctgaggtcaa gttcaactgg tacgtggacg gcgtggaggt gcataatgcc     960

aagacaaagc cgcgggagga gcagtacaac agcacgtacc gggtggtcag cgtcctcacc    1020

gtcctgcacc aggactggct gaatggcaag gagtacaagt gcaaggtctc caacaaagcc    1080

ctcccagccc ccatcgagaa aaccatctcc aaagccaaag ggcagccccg agaaccacag    1140

gtgtacaccc tgcccccatc ccgggatgag ctgaccaaga accaggtcag cctgacctgc    1200

ctggtcaaag gcttctatcc cagcgacatc gccgtggagt gggagagcaa tgggcagccg    1260

gagaacaact acaagaccac gcctcccgtg ctggactccg acggctcctt cttcctctac    1320

agcaagctca ccgtggacaa gagcaggtgg cagcagggga acgtcttctc atgctccgtg    1380

atgcatgagg ctctgcacaa ccactacacg cagaagagcc tctccctgtc tccgggtaaa    1440

taa                                                                  1443

<210> 31
<211> 717
<212> DNA
<213> Artificial Sequence

<220>
<223> Chimeric anti-PrP mAb L chain

<400> 31
atgaagttgc ctgttaggct gttggtgctg atgttctgga ttcctgcttc cagcagtgat 60

gttttgatga cccaaactcc actctccctg cctgtcagtc ttggagatca agcctccatc 120

tcttgcagat ctagtcagag cattgtacat actaatggaa acacctattt agaatggttc 180

ctgcagaaac caggccagtc tcccaagctc ctgatctaca aagtttccaa ccgattttct 240

ggggtcccag acaggttcag tggcagtgga tcagggacag atttcacact caagatcagc 300

agagtggagg ctgaggatct gggagtttat tactgctttc aaggttcact tgttccgtac 360

acgttcggag gggggaccaa gctggaaata aaacgtacgg tggctgcacc atctgtcttc 420

atcttcccgc catctgatga gcagttgaaa tctggaactg cctctgttgt gtgcctgctg 480

aataacttct atcccagaga ggccaaagta cagtggaagg tggataacgc cctccaatcg 540

ggtaactccc aggagagtgt cacagagcag gacagcaagg acagcaccta cagcctcagc 600

agcaccctga cgctgagcaa agcagactac gagaaacaca aagtctacgc ctgcgaagtc 660

acccatcagg gcctgagctc gcccgtcaca aagagcttca acaggggaga gtgttag 717

<210> 32
<211> 1431
<212> DNA
<213> Artificial Sequence

<220>
<223> Chimeric anti-PrP mAb H chain

<400> 32
atgagtattg atcacagacc cctcaccatg aacttcgggc tcagattgat tttccttgtc 60

ctcactttaa aaggtgtcca gtgtgacgtg aagctggtgg agtctgggga aggcttagtg 120

aagcctggag ggtccctgaa actctcctgt gcagcctctg gaatcacttt cagtaggtat 180

gccatgtctt gggttcgcca gactccagag aagaggctgg agtgggtcgc atacattagt 240

agtggtggtg attacatcaa ctatgcagac actgtgaagg gccgattcac catctccaga 300

gacaatgcca ggaacaccct gtacctgcaa atgagcagtc tgaagtctga ggacacagcc 360

atgtattact gtacaagagt aactccatat tggtacttcg atgtctgggg cacagggacc 420

acggtcaccg tctcctcagc gtcgaccaag ggcccatcgg tcttccccct ggcaccctcc 480

tccaagagca cctctggggg cacagcggcc ctgggctgcc tggtcaagga ctacttcccc 540

gaaccggtga cggtgtcgtg gaactcaggc gccctgacca gcggcgtgca caccttcccg 600

gctgtcctac agtcctcagg actctactcc ctcagcagcg tggtgaccgt gccctccagc 660

agcttgggca cccagaccta catctgcaac gtgaatcaca agcccagcaa caccaaggtg 720

gacaagaaag ttgagcccaa atcttgtgac agaactcaca catgcccacc gtgcccagca 780

cctgaactcc tggggggacc gtcagtcttc ctcttccccc caaaacccaa ggacaccctc 840

atgatctccc ggacccctga ggtcacatgc gtggtggtgg acgtgagcca cgaagaccct 900

gaggtcaagt tcaactggta cgtggacggc gtggaggtgc ataatgccaa gacaaagccg 960

cgggaggagc agtacaacag cacgtaccgg gtggtcagcg tcctcaccgt cctgcaccag 1020

gactggctga atggcaagga gtacaagtgc aaggtctcca acaaagccct cccagccccc 1080

atcgagaaaa ccatctccaa agccaaaggg cagccccgag aaccacaggt gtacaccctg 1140

cccccatccc gggatgagct gaccaagaac caggtcagcc tgacctgcct ggtcaaaggc 1200

ttctatccca gcgacatcgc cgtggagtgg gagagcaatg ggcagccgga gaacaactac 1260

aagaccacgc ctcccgtgct ggactccgac ggctccttct tcctctacag caagctcacc 1320

gtggacaaga gcaggtggca gcaggggaac gtcttctcat gctccgtgat gcatgaggct 1380

ctgcacaacc actacacgca gaagagcctc tccctgtctc cgggtaaata a 1431

<210> 33
<211> 717
<212> DNA
<213> Artificial Sequence

<220>
<223> Chimeric anti-PrP mAb L chain

<400> 33
atggagacag acacactcct gctatgggtg ctgctgctct gggttccagg ttccacaggt 60

gacattgtgc tgacccaatc tccagcttct ttgggtgtgt ctctagggca gagggccacc 120

atatcctgca gagccagtga aagtgttgat agttatggca atagttttat gcactggtac 180

cagcagaaac caggacagcc acccaaagtc ctcatctatc gtgcatccaa tcgagaatct 240

gggatccctg ccaggttcag tggcagtggg tctaggacag acttcaccct caccattaat 300

cctgtggagg ctgatgatgt tgcaacctat tactgtcagc aaagtaatga ggatccgtat 360

acattcggag gggggaccaa gctggaaata aaacgtacgg tggctgcacc atctgtcttc 420

atcttcccgc catctgatga gcagttgaaa tctggaactg cctctgttgt gtgcctgctg 480

aataacttct atcccagaga ggccaaagta cagtggaagg tggataacgc cctccaatcg 540

ggtaactccc aggagagtgt cacagagcag gacagcaagg acagcaccta cagcctcagc 600

agcaccctga cgctgagcaa agcagactac gagaaacaca aagtctacgc ctgcgaagtc 660

acccatcagg gcctgagctc gcccgtcaca aagagcttca acaggggaga gtgttag 717

<210> 34
<211> 1395
<212> DNA
<213> Artificial Sequence

<220>
<223> Chimeric anti-PrP mAb H chain

<400> 34
atggaatgga tctggatctt tctcttcatc ctgtcaggaa ctgcaggtgt ccaatcccag 60

gttcagctgc tgcagtctgg agctgaactg gcgaggcctg gggcttcagt gaagctgtcc 120

tgcaagggtt ctggctacac cttcacaagc tatagtataa gttgggtgaa gcagagaact 180

ggacagggcc ttgagtggat tggagagatt tatcctagaa gtggtaatac ttactacaat 240

gagaagttca aggacaaggc cacactgact gcagacaaat cctccagcac agcgtacatg 300

gagctccgca gcctgacatc tgaggactct gcggtctatt tctgtgcaac ggattacctg 360

tttgcttact ggggccaagg gactctggtc actgtctctg cagcgtcgac caagggccca 420

tcggtcttcc ccctggcacc ctcctccaag agcacctctg ggggcacagc ggccctgggc 480

tgcctggtca aggactactt ccccgaaccg gtgacggtgt cgtggaactc aggcgccctg 540

accagcggcg tgcacacctt cccggctgtc ctacagtcct caggactcta ctccctcagc 600

agcgtggtga ccgtgccctc cagcagcttg ggcacccaga cctacatctg caacgtgaat 660

cacaagccca gcaacaccaa ggtggacaag aaagttgagc ccaaatcttg tgacagaact 720

cacacatgcc caccgtgccc agcacctgaa ctcctggggg gaccgtcagt cttcctcttc 780

cccccaaaac ccaaggacac cctcatgatc tcccggaccc ctgaggtcac atgcgtggtg 840

gtggacgtga gccacgaaga ccctgaggtc aagttcaact ggtacgtgga cggcgtggag 900

gtgcataatg ccaagacaaa gccgcgggag gagcagtaca acagcacgta ccgggtggtc 960

agcgtcctca ccgtcctgca ccaggactgg ctgaatggca aggagtacaa gtgcaaggtc 1020

tccaacaaag ccctcccagc ccccatcgag aaaaccatct ccaaagccaa agggcagccc 1080

cgagaaccac aggtgtacac cctgccccca tcccgggatg agctgaccaa gaaccaggtc 1140

agcctgacct gcctggtcaa aggcttctat cccagcgaca tcgccgtgga gtgggagagc 1200

aatgggcagc cggagaacaa ctacaagacc acgcctcccg tgctggactc cgacggctcc 1260

ttcttcctct acagcaagct caccgtggac aagagcaggt ggcagcaggg gaacgtcttc 1320

tcatgctccg tgatgcatga ggctctgcac aaccactaca cgcagaagag cctctccctg 1380

tctccgggta aataa 1395

<210> 35
<211> 711
<212> DNA
<213> Artificial Sequence

<220>
<223> Chimeric anti-PrP mAb L chain

<400> 35
atggacatga gggctcctgc acagattttt ggcttcttgt tgctcttgtt tccaggtacc 60

agatgtgaca tccagatgac ccagtctcca tcctccttat ctgcctctct gggagaaaga 120

gtcagtctca cttgtcgggc aagtcaggac attggtagta gtttaaactg gcttcaacag 180

gaaccagatg gaactattaa acgcctgatc tacgccacat ccagtttaga ttctggtgtc 240

cccaaaaggt tcagtggcag taggtctggg tcagattatt ctctcaccat cagcagcctt 300

gagtctgaag attttgtaga ctattactgt ctgcaatatg caaaatctcc gtacacgttc 360

ggagggggga ccaagctgga aataaaacgt acggtggctg caccatctgt cttcatcttc 420

ccgccatctg atgagcagtt gaaatctgga actgcctctg ttgtgtgcct gctgaataac 480

ttctatccca gagaggccaa agtacagtgg aaggtggata acgccctcca atcgggtaac 540

tcccaggaga gtgtcacaga gcaggacagc aaggacagca cctacagcct cagcagcacc 600

ctgacgctga gcaaagcaga ctacgagaaa cacaaagtct acgcctgcga agtcacccat 660

cagggcctga gctcgcccgt cacaaagagc ttcaacaggg gagagtgtta g 711

<210> 36
<211> 480
<212> PRT
<213> Artificial Sequence

<220>
<223> Chimeric anti-PrP mAb H chain

<400> 36

Met Ser Ser Pro Gln Ala Leu Asn Thr Leu Thr Leu Thr Met Gly Trp
1               5                   10                  15

Ser Trp Ile Phe Leu Leu Phe Leu Ser Gly Thr Ala Gly Val Leu Ser
            20                  25                  30

Glu Val Gln Leu Gln Gln Ser Gly Pro Glu Val Val Lys Pro Gly Ala
        35                  40                  45

Ser Leu Lys Ile Pro Cys Lys Ala Ser Gly Tyr Thr Phe Thr Asp Tyr
    50                  55                  60

Asn Met Asp Trp Val Lys Gln Ser His Gly Lys Ser Leu Glu Trp Ile
65                  70                  75                  80

Gly Asp Ile Asn Pro Asn Asn Gly Gly Thr Ile Tyr Asn His Asn Phe
                85                  90                  95

Thr Asp Lys Ala Thr Leu Thr Val Asp Lys Ser Ser Ser Thr Ala Tyr
            100                 105                 110

Met Glu Leu Arg Ser Leu Thr Ser Glu Asp Thr Ala Val Tyr Tyr Cys
        115                 120                 125

Ala Arg Ala Thr Ser Leu Val Asp Phe Asp Tyr Trp Gly Gln Gly Thr
    130                 135                 140

Thr Leu Thr Val Ser Ser Ala Ser Thr Lys Gly Pro Ser Val Phe Pro
145                 150                 155                 160

Leu Ala Pro Ser Ser Lys Ser Thr Ser Gly Gly Thr Ala Ala Leu Gly
                165                 170                 175

Cys Leu Val Lys Asp Tyr Phe Pro Glu Pro Val Thr Val Ser Trp Asn
            180                 185                 190

Ser Gly Ala Leu Thr Ser Gly Val His Thr Phe Pro Ala Val Leu Gln
        195                 200                 205

Ser Ser Gly Leu Tyr Ser Leu Ser Ser Val Val Thr Val Pro Ser Ser
    210                 215                 220

Ser Leu Gly Thr Gln Thr Tyr Ile Cys Asn Val Asn His Lys Pro Ser
225                 230                 235                 240

Asn Thr Lys Val Asp Lys Lys Val Glu Pro Lys Ser Cys Asp Arg Thr
                245                 250                 255

His Thr Cys Pro Pro Cys Pro Ala Pro Glu Leu Leu Gly Gly Pro Ser
            260                 265                 270

Val Phe Leu Phe Pro Pro Lys Pro Lys Asp Thr Leu Met Ile Ser Arg
        275                 280                 285

Thr Pro Glu Val Thr Cys Val Val Val Asp Val Ser His Glu Asp Pro
    290                 295                 300

Glu Val Lys Phe Asn Trp Tyr Val Asp Gly Val Glu Val His Asn Ala
305                 310                 315                 320

Lys Thr Lys Pro Arg Glu Glu Gln Tyr Asn Ser Thr Tyr Arg Val Val
                325                 330                 335

Ser Val Leu Thr Val Leu His Gln Asp Trp Leu Asn Gly Lys Glu Tyr
            340                 345                 350

Lys Cys Lys Val Ser Asn Lys Ala Leu Pro Ala Pro Ile Glu Lys Thr
        355                 360                 365

Ile Ser Lys Ala Lys Gly Gln Pro Arg Glu Pro Gln Val Tyr Thr Leu
    370                 375                 380

Pro Pro Ser Arg Asp Glu Leu Thr Lys Asn Gln Val Ser Leu Thr Cys
385                 390                 395                 400

Leu Val Lys Gly Phe Tyr Pro Ser Asp Ile Ala Val Glu Trp Glu Ser
                405                 410                 415

Asn Gly Gln Pro Glu Asn Asn Tyr Lys Thr Thr Pro Pro Val Leu Asp
            420                 425                 430

Ser Asp Gly Ser Phe Phe Leu Tyr Ser Lys Leu Thr Val Asp Lys Ser
        435                 440                 445

Arg Trp Gln Gln Gly Asn Val Phe Ser Cys Ser Val Met His Glu Ala
    450                 455                 460

Leu His Asn His Tyr Thr Gln Lys Ser Leu Ser Leu Ser Pro Gly Lys
465                 470                 475                 480

<210>  37
<211>  238
<212>  PRT
<213>  Artificial Sequence

<220>
<223>  Chimeric anti-PrP mAb L chain

<400>  37

Met Lys Leu Pro Val Arg Leu Leu Val Leu Met Phe Trp Ile Pro Ala
1               5                   10                  15

Ser Ser Ser Asp Val Leu Met Thr Gln Thr Pro Leu Ser Leu Pro Val
            20                  25                  30

Ser Leu Gly Asp Gln Ala Ser Ile Ser Cys Arg Ser Ser Gln Ser Ile
        35                  40                  45

Val His Thr Asn Gly Asn Thr Tyr Leu Glu Trp Phe Leu Gln Lys Pro
    50                  55                  60

Gly Gln Ser Pro Lys Leu Leu Ile Tyr Lys Val Ser Asn Arg Phe Ser
65                  70                  75                  80

Gly Val Pro Asp Arg Phe Ser Gly Ser Gly Ser Gly Thr Asp Phe Thr
                85                  90                  95

Leu Lys Ile Ser Arg Val Glu Ala Glu Asp Leu Gly Val Tyr Tyr Cys
            100                 105                 110

Phe Gln Gly Ser Leu Val Pro Tyr Thr Phe Gly Gly Gly Thr Lys Leu
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  38
<211>  476
<212>  PRT
<213>  Artificial Sequence

<220>
<223>  Chimeric anti-PrP mAb H chain

<400>  38

Met Ser Ile Asp His Arg Pro Leu Thr Met Asn Phe Gly Leu Arg Leu
1               5                   10                  15

Ile Phe Leu Val Leu Thr Leu Lys Gly Val Gln Cys Asp Val Lys Leu
            20                  25                  30

Val Glu Ser Gly Glu Gly Leu Val Lys Pro Gly Gly Ser Leu Lys Leu
        35                  40                  45

Ser Cys Ala Ala Ser Gly Ile Thr Phe Ser Arg Tyr Ala Met Ser Trp
    50                  55                  60

Val Arg Gln Thr Pro Glu Lys Arg Leu Glu Trp Val Ala Tyr Ile Ser
65                  70                  75                  80

Ser Gly Gly Asp Tyr Ile Asn Tyr Ala Asp Thr Val Lys Gly Arg Phe
                85                  90                  95

Thr Ile Ser Arg Asp Asn Ala Arg Asn Thr Leu Tyr Leu Gln Met Ser
            100                 105                 110

Ser Leu Lys Ser Glu Asp Thr Ala Met Tyr Tyr Cys Thr Arg Val Thr
        115                 120                 125

Pro Tyr Trp Tyr Phe Asp Val Trp Gly Thr Gly Thr Thr Val Thr Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Leu Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220

Gln Thr Tyr Ile Cys Asn Val Asn His Lys Pro Ser Asn Thr Lys Val

225                 230                 235                 240

Asp Lys Lys Val Glu Pro Lys Ser Cys Asp Arg Thr His Thr Cys Pro
                245                 250                 255

Pro Cys Pro Ala Pro Glu Leu Leu Gly Gly Pro Ser Val Phe Leu Phe
            260                 265                 270

Pro Pro Lys Pro Lys Asp Thr Leu Met Ile Ser Arg Thr Pro Glu Val
        275                 280                 285

Thr Cys Val Val Val Asp Val Ser His Glu Asp Pro Glu Val Lys Phe
    290                 295                 300

Asn Trp Tyr Val Asp Gly Val Glu Val His Asn Ala Lys Thr Lys Pro
305                 310                 315                 320

Arg Glu Glu Gln Tyr Asn Ser Thr Tyr Arg Val Val Ser Val Leu Thr
                325                 330                 335

Val Leu His Gln Asp Trp Leu Asn Gly Lys Glu Tyr Lys Cys Lys Val
            340                 345                 350

Ser Asn Lys Ala Leu Pro Ala Pro Ile Glu Lys Thr Ile Ser Lys Ala
        355                 360                 365

Lys Gly Gln Pro Arg Glu Pro Gln Val Tyr Thr Leu Pro Pro Ser Arg
    370                 375                 380

Asp Glu Leu Thr Lys Asn Gln Val Ser Leu Thr Cys Leu Val Lys Gly
385                 390                 395                 400

Phe Tyr Pro Ser Asp Ile Ala Val Glu Trp Glu Ser Asn Gly Gln Pro
                405                 410                 415

Glu Asn Asn Tyr Lys Thr Thr Pro Pro Val Leu Asp Ser Asp Gly Ser
            420                 425                 430

Phe Phe Leu Tyr Ser Lys Leu Thr Val Asp Lys Ser Arg Trp Gln Gln
        435                 440                 445

Gly Asn Val Phe Ser Cys Ser Val Met His Glu Ala Leu His Asn His
    450                 455                 460

Tyr Thr Gln Lys Ser Leu Ser Leu Ser Pro Gly Lys
465                 470                 475

<210>  39
<211>  238
<212>  PRT
<213>  Artificial Sequence

<220>
<223>  Chimeric anti-PrP mAb L chain

<400>  39

Met Glu Thr Asp Thr Leu Leu Leu Trp Val Leu Leu Leu Trp Val Pro
1               5                   10                  15

Gly Ser Thr Gly Asp Ile Val Leu Thr Gln Ser Pro Ala Ser Leu Gly
            20                  25                  30

Val Ser Leu Gly Gln Arg Ala Thr Ile Ser Cys Arg Ala Ser Glu Ser
        35                  40                  45

Val Asp Ser Tyr Gly Asn Ser Phe Met His Trp Tyr Gln Gln Lys Pro
    50                  55                  60

Gly Gln Pro Pro Lys Val Leu Ile Tyr Arg Ala Ser Asn Arg Glu Ser
65                  70                  75                  80

Gly Ile Pro Ala Arg Phe Ser Gly Ser Gly Ser Arg Thr Asp Phe Thr
                85                  90                  95

Leu Thr Ile Asn Pro Val Glu Ala Asp Asp Val Ala Thr Tyr Tyr Cys
            100                 105                 110

Gln Gln Ser Asn Glu Asp Pro Tyr Thr Phe Gly Gly Gly Thr Lys Leu
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  40
<211>  464
<212>  PRT
<213>  Artificial Sequence

<220>
<223>  Chimeric anti-PrP mAb H chain

<400>  40

Met Glu Trp Ile Trp Ile Phe Leu Phe Ile Leu Ser Gly Thr Ala Gly
1               5                   10                  15

Val Gln Ser Gln Val Gln Leu Leu Gln Ser Gly Ala Glu Leu Ala Arg
            20                  25                  30

Pro Gly Ala Ser Val Lys Leu Ser Cys Lys Gly Ser Gly Tyr Thr Phe
        35                  40                  45

Thr Ser Tyr Ser Ile Ser Trp Val Lys Gln Arg Thr Gly Gln Gly Leu
    50                  55                  60

Glu Trp Ile Gly Glu Ile Tyr Pro Arg Ser Gly Asn Thr Tyr Tyr Asn
65                  70                  75                  80

Glu Lys Phe Lys Asp Lys Ala Thr Leu Thr Ala Asp Lys Ser Ser Ser
                85                  90                  95

Thr Ala Tyr Met Glu Leu Arg Ser Leu Thr Ser Glu Asp Ser Ala Val
            100                 105                 110

Tyr Phe Cys Ala Thr Asp Tyr Leu Phe Ala Tyr Trp Gly Gln Gly Thr
        115                 120                 125

Leu Val Thr Val Ser Ala Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
                165                 170                 175

Ser Gly Ala Leu Thr Ser Gly Val His Thr Phe Pro Ala Val Leu Gln
            180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Lys Val Glu Pro Lys Ser Cys Asp Arg Thr
225                 230                 235                 240

His Thr Cys Pro Pro Cys Pro Ala Pro Glu Leu Leu Gly Gly Pro Ser
                245                 250                 255

Val Phe Leu Phe Pro Pro Lys Pro Lys Asp Thr Leu Met Ile Ser Arg
            260                 265                 270

Thr Pro Glu Val Thr Cys Val Val Val Asp Val Ser His Glu Asp Pro
        275                 280                 285

Glu Val Lys Phe Asn Trp Tyr Val Asp Gly Val Glu Val His Asn Ala
    290                 295                 300

Lys Thr Lys Pro Arg Glu Glu Gln Tyr Asn Ser Thr Tyr Arg Val Val
305                 310                 315                 320

Ser Val Leu Thr Val Leu His Gln Asp Trp Leu Asn Gly Lys Glu Tyr
                325                 330                 335

Lys Cys Lys Val Ser Asn Lys Ala Leu Pro Ala Pro Ile Glu Lys Thr
            340                 345                 350

Ile Ser Lys Ala Lys Gly Gln Pro Arg Glu Pro Gln Val Tyr Thr Leu
        355                 360                 365

Pro Pro Ser Arg Asp Glu Leu Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400

Asn Gly Gln Pro Glu Asn Asn Tyr Lys Thr Thr Pro Pro Val Leu Asp
                405                 410                 415

Ser Asp Gly Ser Phe Phe Leu Tyr Ser Lys Leu Thr Val Asp Lys Ser
            420                 425                 430

Arg Trp Gln Gln Gly Asn Val Phe Ser Cys Ser Val Met His Glu Ala
        435                 440                 445

Leu His Asn His Tyr Thr Gln Lys Ser Leu Ser Leu Ser Pro Gly Lys
    450                 455                 460

<210> 41
<211> 236
<212> PRT
<213> Artificial Sequence

<220>
<223> Chimeric anti-PrP mAb L chain

<400> 41

Met Asp Met Arg Ala Pro Ala Gln Ile Phe Gly Phe Leu Leu Leu Leu
1               5                   10                  15

Phe Pro Gly Thr Arg Cys Asp Ile Gln Met Thr Gln Ser Pro Ser Ser
            20                  25                  30

Leu Ser Ala Ser Leu Gly Glu Arg Val Ser Leu Thr Cys Arg Ala Ser
        35                  40                  45

Gln Asp Ile Gly Ser Ser Leu Asn Trp Leu Gln Gln Glu Pro Asp Gly
    50                  55                  60

Thr Ile Lys Arg Leu Ile Tyr Ala Thr Ser Ser Leu Asp Ser Gly Val
65                  70                  75                  80

Pro Lys Arg Phe Ser Gly Ser Arg Ser Gly Ser Asp Tyr Ser Leu Thr
                85                  90                  95

Ile Ser Ser Leu Glu Ser Glu Asp Phe Val Asp Tyr Tyr Cys Leu Gln
            100                 105                 110

Tyr Ala Lys Ser Pro Tyr Thr Phe Gly Gly Gly Thr Lys Leu Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235

## Description

### TECHNICAL FIELD

The present invention relates to a remedy for a prion disease, the remedy containing as an effective component mesenchymal cells and, in particular, mesenchymal cells having abnormal prion growth inhibitory activity, and a method for producing it. Furthermore, the present invention relates to cells into which a gene having abnormal prion growth inhibitory activity is introduced and, in particular, mesenchymal cells, and a method for producing same. Moreover, the present invention relates to a method for treating a prion disease using the above-mentioned remedy and/or cells, or an anti-prion antibody. Furthermore, the present invention relates to an agent for delivering a substance to a lesion site of a prion disease by the use of mesenchymal cells.

### BACKGROUND ART

Recently, prion disease caused by the accumulation of abnormal prion protein (PrP^{Sc}) has received much attention. Prion disease is seen in various species of animal, and human kuru, Creutzfeldt-Jakob disease (CJD), and Gerstmann-Straussler-Scheinker disease (GSS), sheep and goat scrapie, mink transmissible encephalopathy, deer chronic wasting disease, bovine spongiform encephalopathy (BSE), feline spongiform encephalopathy, etc. are known. In all of these there are the common features that the incubation period up to onset is long, brain atrophy accompanied by neuronal loss is observed, and various neurological symptoms are presented.

Prion protein is protein that is usually present on the surface of a normal cell. Normal prion protein (PrP^{c}) and PrP^{Sc} have an identical amino acid sequence but a different conformation, and as a result the two have completely different biological and physical properties. At present, a hypothesis that PrP^{c} changes into PrP^{Sc} upon contact with PrP^{Sc} and, as a result, PrP^{Sc} grows and modifies neurons to cause a prion disease is plausible, but the precise mechanism is as yet unclear. A prion disease has the property of infecting by crossing an interspecies barrier as a result of ingestion or administration of PrP^{Sc} and, among others, human infection with BSE has become a serious problem in recent years.

The human body is infected with BSE as a result of ingestion, etc. of a specific bovine site containing PrP^{Sc}, thus causing variant Creutzfeldt-Jakob disease (vCJD). vCJD is a disease in which neurological manifestations and disorders of higher functions (deterioration of memory, deterioration in calculating ability, disorientation, behavioral abnormalities, change in character, indifference, anxiety, insomnia, agnosia, hallucinations, etc.) occur first, dementia, delusions, and apraxia rapidly progress in a few months, furthermore, standing up and walking become impossible, akinetic mutism occurs in 3 to 7 months, and death is caused by general prostration, respiratory paralysis, pneumonia, etc. in 1 to 2 years. There have been 154 reported cases worldwide of vCJD due to infection with BSE up to March 2005, the first infected Japanese person was reported on February 4, 2005, and the influence of vCJD is gradually widening. According to a trend prediction, it is estimated that a few thousand to a few tens of thousands of patients will manifest symptoms in the near future, and it is expected that there will be at least a few tens of thousands of patients per year in Japan in the future.

In such circumstances, a broad range of research into the treatment of prion disease has been carried out, but the current situation is that no effective treatment method has yet been established.
In initial research, attempts to search for an effective agent for prion disease from among known compounds were made; it has been suggested from the results of cultured cell or animal experiments that, among them, amphotericin B (Xi YG et al. 1992. Nature 356(6370): 598-601), Congo red (Caughey B et al. 1992. J Neurochem 59(2):768-71), anthracycline (Tagliavini F et al. 1997. Science 276(5315): 1119-22), cysteine protease inhibitors such as quinacrine, tilorone, chloroquine, and E-64d (Doh-Ura K et al. 2000. J Virol 74(10): 4894-7), phenothiazine derivatives such as promazine, chlorpromazine, and acepromazine (Korth C et al. 2001. Proc Natl Acad Sci USA 98(17): 9836-41), tetrapyrroles such as porphyrin and phthalocyanine (Caughey WS et al. 1998. Proc Natl Acad Sci USA 95(21): 12117-22), pentosan polysulfate, reactive dyes such as reactive green and reactive red, quinines such as quinine, 8-hydroxyquinoline-2-carboxaldehyde 8-quinolylhydrazone, 2-pyridinecarboxaldehyde 2-quinolylhydrazone, and 2,2'-biquinoline (JP, A, 2003-40778), etc. might be effective, but the usefulness thereof has not yet been established.

In Patent Publication 1 it is stated that, noting that the expression of PrP^{c}, and consequently PrP^{Sc}, can be suppressed by decreasing the acidic isoform of GAPDH and/or increasing the basic isoform thereof, it is possible to prevent and treat a prion disease by the administration of an antisense nucleic acid of GAPDH or deprenyl. However, in such a method, there is a possibility that, although the generation of PrP^{Sc} might be reduced, the expression of PrP^{c} is suppressed at the same time, and there might be a problem with achieving the original function of PrP^{c}.

Non-patent Publication 1 states that the formation of PrP^{Sc} is inhibited in vitro by a mutant of PrP^{c} by the dominant negative effect, suggesting its usefulness in the treatment of a prion disease, but its usefulness in vivo has not yet been confirmed.
Furthermore, it has been reported recently that an anti-prion antibody inhibits the formation of PrP^{Sc} in vitro and also has an effect in reducing PrP^{Sc} and an effect in prolonging life in a scrapie model mouse in vivo (Non-patent Publication 2); an antibody therapy for a prion disease has attracted attention, but it has subsequently been reported that administration of an anti-prion antibody has caused apoptosis in neurons of the hippocampus and the cerebellum (Non-patent Publication 3), and its usefulness is still unclear.

As hereinbefore described, in spite of intensive world-wide research being carried out, no treatment method that is effective for a prion disease currently exists, and there has been a strong desire for new remedies and treatment methods.
[Patent Publication 1] JP, A, 2004-81034
[Patent Publication 2] WO 03/038074
[Non-patent Publication 1] Kaneko K et al. 1997. Proc Natl Acad Sci USA. 94(19): 10069-74
[Non-patent Publication 2] White AR et al. 2003. Nature 422(6927): 80-3
[Non-patent Publication 3] Solforosi L et al. 2004. Science 303(5663): 1514-6

### DISCLOSURE OF THE INVENTION

### PROBLEMS TO BE SOLVED BY THE INVENTION

It is an object of the present invention to provide a drug that is effective for the treatment of a prion disease and is very safe.

### MEANS FOR SOLVING THE PROBLEMS

As a result of an intensive investigation by the present inventors in order to solve the above-mentioned problems, it has been found that by continuously administering an anti-prion antibody to a prion disease model mouse, a remarkable therapeutic effect can be obtained. Furthermore, it has been found that by introducing a gene that codes for the above-mentioned antibody into myeloid stem cells, said gene is successfully expressed and the myeloid stem cells survive in an affected region, and the present invention has thus been accomplished.

That is, the present invention relates to an agent for the treatment of a prion disease, the agent comprising mesenchymal cells as an effective component.
Furthermore, the present invention relates to the agent wherein the mesenchymal cells have abnormal prion growth inhibitory activity.
Moreover, the present invention relates to the agent wherein the mesenchymal cells have introduced thereinto an anti-prion antibody gene having abnormal prion growth inhibitory activity.
Furthermore, the present invention relates to the agent, wherein the antibody gene comprises an antibody heavy chain gene and an antibody light chain gene,
the antibody heavy chain gene being selected from the group consisting of
(1a) a nucleic acid that contains a sequence selected from the group consisting of SEQ ID NOS: 1, 3, 5, 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for, the antibody light chain gene being selected from the group consisting of
(2a) a nucleic acid containing a sequence selected from the group consisting of SEQ ID NOS: 2, 4, 6, 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for, and
an antibody obtained by expression of the antibody heavy chain gene and the antibody light chain gene having abnormal prion growth inhibitory activity.

Moreover, the present invention relates to the agent, wherein it is for intravenous administration.
Furthermore, the present invention relates to the agent, wherein the mesenchymal cells are selected from the group consisting of bone-marrow cells, umbilical cord blood cells, and peripheral blood cells.
Moreover, the present invention relates to an anti-prion antibody gene comprising an antibody heavy chain gene and an antibody light chain gene,
the antibody heavy chain gene being selected from the group consisting of
(1a) a nucleic acid that contains a sequence selected from the group consisting of SEQ ID NOS: 1, 3, 5, 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for,
   the antibody light chain gene being selected from the group consisting of
(2a) a nucleic acid containing a sequence selected from the group consisting of SEQ ID NOS: 2, 4, 6, 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for, and an antibody obtained by expression of the antibody heavy chain gene and the antibody light chain gene having abnormal prion growth inhibitory activity.

Furthermore, the present invention relates to a vector comprising the anti-prion antibody gene.
Moreover, the present invention relates to the vector, wherein it is an adenovirus vector containing an RGD sequence.
Furthermore, the present invention relates to an anti-prion chimera antibody comprising an antibody variable region that is coded for by the anti-prion antibody gene, and an antibody constant region of an animal other than a mouse.
Moreover, the present invention relates to the anti-prion chimera antibody, wherein it is coded for by
an antibody heavy chain gene selected from the group consisting of
(1a) a nucleic acid that contains a sequence selected from the group consisting of SEQ ID NOS: 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for, and
   an antibody light chain gene selected from the group consisting of
(2a) a nucleic acid containing a sequence selected from the group consisting of SEQ ID NOS: 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for.

Furthermore, the present invention relates to a nucleic acid that codes for the anti-prion chimera antibody.
Moreover, the present invention relates to a method of producing a cells having abnormal prion growth inhibitory activity, the method comprising introducing into cells a gene that imparts abnormal prion growth inhibitory activity.
Furthermore, the present invention relates to the method, wherein the gene that imparts abnormal prion growth inhibitory activity is an anti-prion antibody gene.
Moreover, the present invention relates to a method of producing cells having abnormal prion growth inhibitory activity, the method comprising introducing the gene into cells via the vector.
Furthermore, the present invention relates to the method, wherein the cells are mesenchymal cells.
Moreover, the present invention relates to cells, produced by the method, having abnormal prion growth inhibitory activity.
Furthermore, the present invention relates to a method of producing an agent for the treatment of a prion disease, the method comprising the above-mentioned method.

Moreover, the present invention relates to a sustained-release preparation for the treatment of a prion disease, the preparation releasing an anti-prion antibody.
Furthermore, the present invention relates to the preparation, wherein the anti-prion antibody is an antibody that is coded for by the anti-prion antibody gene and/or the anti-prion chimera antibody.
Moreover, the present invention relates to the preparation, wherein it is in osmotic pump form.
Furthermore, the present invention relates to the preparation, wherein it comprises anti-prion antibody-secreting cells.
Moreover, the present invention relates to the preparation, wherein the anti-prion antibody-secreting cells are cells, produced by the above-mentioned method, having abnormal prion growth inhibitory activity.
Furthermore, the present invention relates to a method of treating a prion disease, the method comprising administering a therapeutically effective amount of a remedy selected from the group consisting of the above-mentioned agent, vector, anti-prion chimera antibody, and preparation.
Moreover, the present invention relates to a method of treating a prion disease, the method comprising sustainedly releasing an anti-prion antibody.
Furthermore, the present invention relates to the method, wherein it comprises administering the sustained-release preparation and/or vector.
Moreover, the present invention relates to the method, wherein it comprises introducing an anti-prion antibody gene into target cells.
Furthermore, the present invention relates to the use of mesenchymal cells in producing an agent for delivering a substance to a lesion site of a prion disease.
Moreover, the present invention relates to an agent for delivering a substance to a lesion site of a prion disease, the agent comprising mesenchymal cells.

### EFFECTS OF THE INVENTION

By the administration of the remedy or the treatment method of the present invention, it becomes possible to treat a prion disease, which had been thought to be impossible, and a great contribution to medical and veterinary fields can be expected. Moreover, since mesenchymal cells (mesenchymal stem cells), which are used in the agent of the present invention, can be produced from bone marrow, umbilical cord blood, or peripheral blood, no ethical concerns arise, unlike a case in which ES cells are used and, furthermore, since production from self-derived cells is easy, there is little possibility of causing an undesirable biological reaction such as a rejection response when administered. Furthermore, although a conventional remedy principally only has PrP^{Sc} growth inhibitory activity and can only delay the progression of symptoms, the agent of the present invention can exhibit, in addition to such activity, excellent effects in not only delaying the progression of symptoms but also in improving the symptoms, which could never be achieved by the conventional remedy, since mesenchymal cells that have migrated to an affected area become fixed thereto and themselves differentiate into neurons to thus reconstruct denatured nerve tissue. Moreover, since the delivery agent of the present invention can deliver a desired substance such as a remedy or a labeled substance to a lesion site of a prion disease, it greatly contributes not only to the treatment of a prion disease but also to research and diagnosis thereof. The above-mentioned effects of the present invention can easily be confirmed by, for example, administering the remedy of the present invention to a prion disease model mouse.

### BRIEF DESCRIPTION OF DRAWINGS

[FIG. 1] A schematic diagram showing the positional relationship between an antibody gene and a primer.
[FIG. 2] A schematic diagram showing a procedure of cloning an antibody gene into an expression vector.
[FIG. 3] A diagram showing the result of evaluation by a Western blot method of the expression of an antibody by antibody gene-transfected 293T cells.
[FIG. 4] A graph showing the ability of an antibody produced by the antibody gene-transfected 293T cells to bind to a prion protein.
[FIG. 5] A schematic diagram of a cosmid containing an antibody gene.
[FIG. 6] A diagram showing the results of FACS analysis carried out for CD45+ cells and CD45- cells.

[FIG. 7] A photographic diagram showing the morphological difference between CD45+ cells and the CD45-cells.
[FIG. 8] A diagram showing the result of evaluation by a Western blot method of the expression of an antibody gene by ICR mouse MSC simultaneously infected with adenovirus vectors Ax43C5H and Ax43C5L containing 43C5 antibody gene. Lane 1 is a control, and Lane 2 is cells with antibody gene introduced thereinto.
[FIG. 9] A photographic diagram showing an isotype of an antibody produced by human and mouse MSC into which has been introduced an expression vector (pCAcc/72-5H and pCAcc/72-5L) containing 72-5 antibody gene or an expression vector (pCAcc/44B1H and pCAcc/44B1L) containing 44B1 antibody gene.
[FIG. 10] A photographic diagram showing the result of staining, with X-gal, ICR/MSC into which a LacZ gene has been introduced.
[FIG. 11] A diagram showing the result of FACS analyses carried out for human MSC or ICR/MSC into which a DsRed gene has been introduced.

[FIG. 12] A photographic diagram showing the state of brain tissue after 72-5 antibody or 44B1 antibody has been intracerebrally administered to a prion disease model mouse for a predetermined period. KLH was used as a negative control.
[FIG. 13] A diagram comparing the amount of PrP^{Sc} accumulated after 72-5 antibody or 44B1 antibody was intracerebrally administered to a prion disease model mouse for a predetermined period, with one to which KLH was administered as a negative control. Figures in the drawing denote the ratio of the amount of PrP^{Sc} accumulated in the subject to which the antibody was administered when the figure with KLH is defined as 1.00.
[FIG. 14] A photographic diagram showing the state after transplanting LacZ-expressing MSC into a prion disease model mouse (infected mouse) or a normal ICR mouse (noninfected mouse).

### BEST MODE FOR CARRYING OUT THE INVENTION

Unless otherwise stated in the present specification, scientific and technical terms used with respect to the present invention have the meaning normally understood by a person skilled in the art. In general, terms and techniques used with respect to cell and tissue culturing, molecular biology, immunology, microbiology, gene, protein, and nucleic acid chemistry, and hybridization described in the present specification are well known in the art and normally used. In general, unless otherwise stated, the methods and techniques of the present invention are carried out in accordance with standard methods well known in the art and as described in various general and specialized publications referred to or discussed in the present specification. As such publications, for example, there can be cited Sambrook et al., Molecular Cloning: A Laboratory Manual, 2d ed., Cold Spring Harbor Laboratory Press (1989) and Sambrook et al., Molecular Cloning: A Laboratory Manual, 3d ed., Cold Spring Harbor Press (2001); Ausubel et al., Current Protocols in Molecular Biology, Greene Publishing Associates (1992, and supplement in 2000); Ausubel et al., Short Protocols in Molecular Biology: A Compendium of Methods from Current Protocols in Molecular Biology - 4th Ed., Wiley & Sons (1999); Harlow and Lane, Antibodies: A Laboratory Manual, Cold Spring Harbor Laboratory Press (1990); and Harlow and Lane, Using Antibodies: A Laboratory Manual, Cold Spring Harbor Laboratory Press (1999), etc.

Enzyme reactions and purification techniques are carried out in accordance with manufacturer-supplied specifications and as normally carried out in the art or as described in the present specification. Terms, experimental procedures, and techniques used with respect to analytical chemistry, synthetic organic chemistry, medicinal chemistry, and pharmaceutical chemistry described in the present specification are well known in the art and normally used. Standard techniques are employed in chemical synthesis, chemical analysis, production, formulation, and delivery of an agent, and treatment of a subject.
The term 'subject' referred to in the present invention means any biological individual, preferably an animal, more preferably a mammal, and yet more preferably a human individual. In the present invention, the subject may be healthy or affected by any disease, but when an attempt is made to treat a prion disease, the subject is typically a subject affected by said disease or an experimentally affected subject, for example, a rodent such as a mouse, a rat, a sand rat, or a guinea pig, an animal of the feline family such as a cat, a puma, or a tiger, a cervid such as a deer or an elk, a mink, a sheep, a goat, a cow, a monkey, man, etc.

In the present invention, there is provided an agent for the treatment of a prion disease, the agent containing mesenchymal cells as an effective component.
The prion disease referred to in the present invention includes all diseases that are caused by PrP^{Sc}. Examples of such diseases include kuru, Creutzfeldt-Jakob disease (CJD), Gerstmann-Straussler-Scheinker disease (GSS), sheep and goat scrapie, mink transmissible encephalopathy, deer chronic wasting disease, bovine spongiform encephalopathy (BSE), and feline spongiform encephalopathy, but are not limited thereto. The above-mentioned diseases may be congenital or acquired (including those that are contagious), and examples thereof include variant CJD (vCJD), which is thought to develop by ingestion of BSE-infected beef, and iatrogenic CJD due to the use of human dried dura mater, etc. The prion disease of the present invention also includes any unknown disease that is caused by PrP^{Sc}.

The mesenchymal cells referred to in the present invention preferably mean bone-marrow cells (a mononuclear cell fractionated component of bone-marrow cells), umbilical cord blood cells, peripheral blood cells, mesenchymal stem cells, cells derived from these cells, etc. The mesenchymal cells of the present invention include, for example, cells related to the mesenchymal system and mesodermal stem cells. In addition, if cells that are described as 'mesenchymal cells' in the present invention are classified as cells other than mesenchymal cells in the future, said cells may suitably be used in the present invention.

The bone marrow includes as stem cells hematopoietic stem cells and mesenchymal stem cells (MSC: Mesenchymal Stem Cells)'. The 'stem cells' referred to here are undifferentiated cells that generally have both an ability to self-propagate and an ability to differentiate into cells having a specific function in a process of physiological growth and differentiation of cells forming a biological body. Hematopoietic stem cells are stem cells that differentiate into red blood cells, white blood cells, or blood platelets. It is known that there is a case in which mesenchymal stem cells differentiate into neurons via neural stem cells, a case in which they differentiate directly into neurons without becoming neural stem cells, a case in which they differentiate into neurons via stroma cells, a case in which they differentiate into an internal organ, a case in which they differentiate into a vascular system, or a case in which they differentiate into bone, cartilage, fat, or muscle.

In the present invention, it is mainly mesenchymal stem cells that are used, but it should be mentioned that there is a possibility that hematopoietic stem cells or other stem cells (progenitor cells) in the body might be used. Mesenchymal stem cells may be obtained by separating them from bone-marrow cells harvested from the bone marrow, etc. In addition, bone-marrow cells from which mesenchymal stem cells have not been separated may be used for the treatment in the same manner as mesenchymal stem cells although the usefulness is slightly inferior.
It might be expected that there would be a possibility that cells like mesenchymal stem cells could be produced from peripheral blood. In practice, the present inventors have successfully induced cells that express markers for neural stem cells or neural cells (neurons, glial cells) from cells cultured from cells present in peripheral blood. On the other hand, the present inventors have already found that, when ES cells or mesodermal stem cells (mesenchymal stem cells) prepared from a mononuclear cell fraction separated from bone marrow fluid or umbilical cord blood are cultured in a standard culture, said ES cells or said mesodermal stem cells (mesenchymal stem cells) are induced to differentiate into neural stem cells, neurons, or glial cells (ref. Patent Publication 2). It is therefore possible to produce cells having the same function as that of mesenchymal stem cells by culturing cells in peripheral blood, and utilize them in the present invention.

The mesodermal stem cells referred to in the present invention mean cells forming tissue that is embryologically classified as the mesoderm, and includes blood cells. The mesodermal stem cell referred to here means a cell that can copy (divide, grow) a cell having the same ability as itself and has the ability to differentiate into all types of cells forming mesodermal tissue. The mesodermal stem cell is a cell having, for example, SH2+, SH3+, SH4+, CD29+, CD44+, CD11b-, CD14-, CD34-, and CD45- features, but is not limited by these markers. Furthermore, the so-called stem cells related to the mesenchymal system are also included in the mesodermal stem cells of the present invention.

The above-mentioned cells related to the mesenchymal system mean mesenchymal stem cells, mesenchymal cells, progenitor cells of mesenchymal cells, and cells derived from mesenchymal cells.
Mesenchymal stem cells mean, for example, stem cells that can be obtained from bone marrow, peripheral blood, skin, hair root, muscular tissue, endometrial membrane, blood, umbilical cord blood and, furthermore, an initial culture of various tissues. Cells that have the same function as that of mesenchymal stem cells that can be obtained by culturing cells in peripheral blood are also included in the mesenchymal stem cells of the present invention.
As preferred mesenchymal cells in the present invention, bone-marrow cells and myeloid stem cells can suitably be cited. In addition, preferred examples of the cells of the present invention include umbilical cord blood cells, peripheral blood cells, and fetal liver cells.

With regard to a preferred embodiment of bone-marrow cells, umbilical cord blood cells, peripheral blood cells, and fetal liver cells in the present invention, a cell fraction that is one fraction of cells obtained by separation from bone marrow, umbilical cord blood, peripheral blood, or fetal liver, and that contains cells that can differentiate into neural cells can be cited.
In another embodiment, said cell fraction is a cell fraction containing mesodermal stem cells having SH2+, SH3+, SH4+, CD29+, CD44+, CD14-, CD34-, and CD45- features.
In the present invention, examples of cell fractions other than the above include cell fractions containing stroma cells having Lin-, Sca-1+, CD10+, CD11D+, CD44+, CD45+, CD71+, CD90+, CD105+, CDW123+, CD127+, CD164+, fibronectin+, ALPH+, and collagenase-1+ features, or a cell fraction containing cells having AC133+ features.
Furthermore, in the present invention, the cells contained in the above-mentioned cell fraction are preferably cells that can differentiate into neural cells.

The cell fraction in the present invention includes cells that are in a mononuclear cell fraction obtained by separation from bone-marrow cells and can differentiate into neural cells. Another embodiment includes cells that are in a mononuclear cell fraction obtained by separation from, for example, umbilical cord blood cells, peripheral blood cells, or fetal liver cells and can differentiate into neural cells. Another embodiment includes cells that are mesenchymal stem cells in bone marrow released into peripheral blood using, for example, an active substance or a drug and can differentiate into neural cells. Although it is not clear whether the differentiation of the cells contained in the cell fraction of the present invention into neural cells is caused by the so-called transformation of blood system cells into neural cells, or is due to differentiation of immature cells, capable of differentiating into neurons, that are present in bone-marrow cells, umbilical cord blood cells, or peripheral blood cells, etc., it is surmised that the cells that differentiate into neural cells are mainly stem cells, that is, self-propagating and multipotential cells. Furthermore, cells that differentiate into neural cells can be stem cells that have differentiated into another germ layer to some degree.

The cells contained in the cell fraction of the present invention do not need to grow by means of a nutritional factor (it is possible to grow by means of a nutritional factor), are easy and highly practical in terms of the development of a nerve autotransplantation technique, and can be said to give great benefit to the healthcare industry. The bone-marrow cells, umbilical cord blood cells, and peripheral blood cells (cell fraction) in the present invention are generally of vertebrate origin. They are preferably of mammal origin (e.g. mouse, rat, rabbit, mink, cat, sheep, goat, cow, deer, pig, dog, monkey, man, etc.), but are not particularly limited.

The cell fraction in the present invention may be prepared by, for example, subjecting bone-marrow cells or umbilical cord blood cells harvested from a vertebrate to density-gradient centrifugation in solution at 2000 rpm for a period of time sufficient for separation according to specific gravity and, after the centrifugation, collecting a cell fraction having a certain specific gravity contained in the specific gravity range of 1.07 to 1.1 g/mL. The 'period of time sufficient for separation according to specific gravity' referred to here means a period of time sufficient for cells to occupy a position according to their specific gravity within the solution for the density-gradient centrifugation, and is usually on the order of 10 to 30 minutes. The specific gravity of the cell fraction to be collected is preferably in the range of 1.07 to 1.08 g/mL, and is for example 1.077 g/mL. The solution for the density-gradient centrifugation may employ a Ficol solution or a Percol solution, but is not limited thereto. It is also possible to prepare umbilical cord blood cells harvested from a vertebrate in the same manner as above and use them as a cell fraction.

As a specific example, bone marrow fluid (5 to 10 µL) harvested from a vertebrate is first mixed with a solution (IL-15 2 mL + Ficol 3 mL), and a mononuclear cell fraction (about 1 mL) is extracted by centrifugation (2000 rpm, 15 minutes). This mononuclear cell fraction is mixed with a culture solution (NPBM 2 mL) in order to wash the cells, and centrifugation (2000 rpm, 15 minutes) is carried out again. Subsequently, after the supernatant is removed, precipitated cells are collected. The harvesting source for the cell fraction of the present invention may be, in addition to a thigh bone, a breast bone or an iliac bone forming the pelvis. Other than these bones, a large bone may be used for harvesting. It is also possible to harvest from bone marrow fluid or umbilical cord blood stored in a bone marrow bank. When umbilical cord blood cells are used, they may be harvested from umbilical cord blood stored in a bone marrow bank.

Another embodiment of the cell fraction of the present invention is a cell fraction that is a mononuclear cell fraction obtained by isolation and purification from bone-marrow cells, umbilical cord blood cells, or peripheral blood cells and that contains mesodermal stem cells (mesenchymal stem cells) that can differentiate into neural cells. The cell fraction containing mesodermal stem cells may be obtained by, for example, selecting cells having the above-mentioned cell surface marker such as SH2 from the above-mentioned cell fraction obtained by centrifugation of the bone-marrow cells, the umbilical cord blood cells, or the peripheral blood cells.

Furthermore, a cell fraction containing mesodermal stem cells (mesenchymal stem cells) that can differentiate into neural cells may be prepared by subjecting bone-marrow cells or umbilical cord blood cells harvested from a vertebrate to density-gradient centrifugation at 900 g in solution for a period of time sufficient for separation according to specific gravity and, after the centrifugation, collecting a cell fraction having a certain specific gravity contained in the specific gravity range of 1.07 to 1.1 g/mL. The 'period of time sufficient for separation according to specific gravity' referred to here means a period of time sufficient for cells to occupy a position according to their specific gravity within the solution for the density-gradient centrifugation, and is usually on the order of 10 to 30 minutes. The specific gravity of the cell fraction to be collected can be varied depending on the type of animal from which the cells are derived (e.g. man, rat, mouse). The solution for the density-gradient centrifugation may employ a Ficol solution or a Percol solution, but is not limited thereto.

As a specific example, bone marrow fluid (25 mL) or umbilical cord blood harvested from a vertebrate is first mixed with the same amount of PBS solution, centrifugation (900 g, 10 minutes) is carried out, and the precipitated cells are mixed with PBS and collected (cell concentration on the order of 4 × 10⁷ cell/mL), thus removing the blood components. Subsequently, 5 mL thereof is mixed with a Percol solution (1.073 g/mL), and centrifugation (900 g, 30 minutes) is carried out, thus extracting a mononuclear cell fraction. The mononuclear cell fraction thus extracted is mixed with a culture solution (DMEM, 10% FBS, 1% antibiotic-antimycotic solution) in order to wash the cells, and centrifugation (2000 rpm, 15 minutes) is carried out. Subsequently, the supernatant after centrifugation is removed, and the precipitated cells are collected and cultured (37°C, 5% CO₂ in air) .

Another embodiment of the cell fraction of the present invention is a cell fraction that is a mononuclear cell fraction obtained by separation from bone-marrow cells or umbilical cord blood cells and that contains stroma cells that can differentiate into neural cells. Stroma cells are cells that have, for example, Lin-, Sca-1+, CD10+, CD11D+, CD44+, CD45+, CD71+, CD90+, CD105+, CDW123+, CD127+, CD164+, fibronectin+, ALPH+, and collagenase-1+ features. The cell fraction containing stroma cells may be obtained by, for example, selecting cells having a cell surface marker such as the above-mentioned Lin from the cell fraction obtained by centrifugation of the bone-marrow cells or the umbilical cord blood cells.

Furthermore, it may be prepared by subjecting bone-marrow cells or umbilical cord blood cells harvested from a vertebrate to density-gradient centrifugation at 800 g in solution for a period of time sufficient for separation according to specific gravity and, after the centrifugation, collecting a cell fraction having a certain specific gravity contained in the specific gravity range of 1.07 to 1.1 g/mL. The 'period of time sufficient for separation according to specific gravity' referred to here means a period of time sufficient for cells to occupy a position according to their specific gravity within the solution for the density-gradient centrifugation, and is usually on the order of 10 to 30 minutes. The specific gravity of the cell fraction to be collected is preferably in the range of 1.07 to 1.08 g/mL, and is for example 1.077 g/mL. The solution for the density-gradient centrifugation may employ a Ficol solution or a Percol solution, but is not limited thereto.

As a specific example, bone marrow fluid or umbilical cord blood harvested from a vertebrate is first mixed with the same amount of a solution (PBS + 2% BSA + 0.6% sodium citrate + 1% penicillin-streptomycin), 5 mL thereof is mixed with Ficol + Paque solution (1.077 g/mL), and centrifugation (800 g, 20 minutes) is carried out, thus extracting a mononuclear cell fraction. This mononuclear cell fraction is mixed with a culture solution (αMEM, 12.5% FBS, 12.5% horse serum, 0.2% i-inositol, 20 mM folic acid, 0.1 mM 2-mercaptoethanol, 2 mM L-glutamine, 1 µM hydrocortisone, 1% antibiotic-antimycotic solution) in order to wash the cells, and centrifugation (2000 rpm, 15 minutes) is carried out. Subsequently, the supernatant after centrifugation is removed, and the precipitated cells are collected and cultured (37°C, 5% CO₂ in air).

Another embodiment of the cell fraction of the present invention is a cell fraction that is a mononuclear cell fraction obtained by separation from bone-marrow cells, umbilical cord blood cells, peripheral blood cells, or fetal liver cells and that contains cells having AC133+ features that can differentiate into neural cells. This cell fraction may be obtained by selecting cells having a cell surface marker of the above-mentioned AC133+ from the cell fraction obtained by centrifugation of the bone-marrow cells, the umbilical cord blood cells, or the peripheral blood cells.
Furthermore, as another embodiment, it may be prepared by subjecting fetal liver cells harvested from a vertebrate to density-gradient centrifugation at 2000 rotation in solution for a period of time sufficient for separation according to specific gravity and, after the centrifugation, collecting a cell fraction having a specific gravity in the range of 1.07 to 1.1 g/mL, and collecting cells having AC133+ features from this cell fraction. The 'period of time sufficient for separation according to specific gravity' referred to here means a period of time sufficient for cells to occupy a position according to their specific gravity within the solution for the density-gradient centrifugation, and is usually on the order of 10 to 30 minutes. The solution for density-gradient centrifugation may employ a Ficol solution or a Percol solution, but is not limited thereto.

As a specific example, liver tissue harvested from a vertebrate is first washed in an L-15 solution, subjected to an enzyme treatment (in a solution containing L-15 + 0.01% DNaseI, 0.25% trypsin, and 0.1% collagenase at 37°C for 30 minutes), and made into single cells by pipetting. These fetal liver cells that have become single cells are subjected to centrifugation. The cells thus obtained are washed, and AC133+ cells are collected from the cells thus washed by utilizing AC133 antibody. This enables cells that can differentiate into neural cells to be prepared from the fetal liver cells. The collection of AC133+ cells by utilizing the antibody may be carried out by utilizing magnetic beads or a cell sorter (FACS, etc.).

After the cell fraction containing these mesodermal stem cells (mesenchymal stem cells), stroma cells, or AC133-positive cells is transplanted to a demyelinated spinal cord region, efficient remyelination occurs. In particular, the cell fraction containing the above-mentioned mesodermal stem cells (mesenchymal stem cells) may survive well and differentiate into neural cells or glial cells even when administered to a prion disease model.
Furthermore, examples of cells, contained in the above-mentioned cell fraction, that can differentiate into neural cells include neural stem cells, mesodermal stem cells (mesenchymal stem cells), stroma cells, and AC133-positive cells contained in the above-mentioned cell fraction, but the cells are not limited to these examples as long as they can differentiate into neural cells.
Moreover, examples of the effective component of the remedy for a prion disease in the present invention include not only bone-marrow cells, umbilical cord blood cells, and peripheral blood cells, but also the above-mentioned cell fraction.

The mesenchymal cells used in the agent of the present invention, for example, the bone-marrow cells, umbilical cord blood cells, or peripheral blood cells, may be used for administration as they are, but said cells may be modified in various ways in order to enhance the therapeutic efficiency. Therefore, in accordance with another aspect of the present invention, there is provided an agent for the treatment of a prion disease, the agent comprising such modified mesenchymal cells as an effective component.
As an example of the above-mentioned modification, introduction of a gene that imparts PrP^{Sc} growth inhibitory activity to the cells can be cited. The PrP^{Sc} growth inhibitory activity referred to here means, for example, an ability to suppress or inhibit an absolute increase of the amount of PrP^{Sc} present in the living body and/or an increase relative to the amount of PrP^{c} present, or to bring about an absolute and/or a relative decrease in the amount of PrP^{Sc} present. Such an effect may be achieved directly or indirectly, but it is preferable not to give any undesirable effect to a living body. Furthermore, the above-mentioned activity preferably involves a high degree of suppression, and most preferably reduces the amount of PrP^{Sc} present.

Examples of a gene that can impart PrP^{Sc} growth inhibitory activity include a gene that codes for a polypeptide having PrP^{Sc} growth inhibitory activity. Such polypeptides include an anti-prion antibody, cadherins such as protocadherin 43 and OB-cadherin-1 (JP, A, 2000-512131), plasminogen, a plasminogen fragment, and a derivative thereof (JP, A, 2004-501626), but are not limited thereto.

In the present invention, the anti-prion antibody referred to means an antibody that is capable of binding to PrP^{c} and/or PrP^{Sc}. In the present specification, unless otherwise specified, the term 'antibody' includes an intact immunoglobulin or an antigen-binding portion thereof. Examples of the antigen-binding portion include Fab, Fab', F(ab')₂, Fv, dAb, and a complementarity determining region fragment, and also includes other portions that are capable of binding to PrP^{c} and/or PrP^{Sc}. The anti-prion antibody used in the present invention preferably has PrP^{Sc} growth inhibitory activity, and examples thereof include those described in Non-patent Publication 2, Non-patent Publication 3, Enari M et al. 2001. Proc Natl Acad Sci USA 98(16): 9295-9, Peretz D et al. 2001. Nature 412(6848): 739-43, Gilch S et al. 2003. J Biol Chem 278(20): 18524-31, etc., and those produced by hybridoma clones 72-5 (Depository No.: FERM P-18516) and 44B1 (Depository No.: FERM P-18515), which were both deposited in the International Patent Organism Depositary, National Institute of Advanced Industrial Science and Technology (Post code 305-8566, Central 6, 1-1-1 Higashi, Tsukuba, Ibaraki, Japan) on September 14, 2001, etc. Furthermore, an anti-prion antibody that does in fact have said activity, but for which the possession of such activity is not known, may suitably be used in the present invention as well. Such an antibody may be screened by an evaluation system employing a scrapie-infected cell line, etc. (ref. Enari M et al., Gilch S et al. above, etc.).

The anti-prion antibody in the present invention includes a chimera antibody, a primatized antibody, and a humanized antibody. Such an antibody has poor immunogenicity in man, and is therefore more suitable than an antibody from a nonhuman animal for in vivo administration to man. The anti-prion chimera antibody was not known until now, has been produced by the present inventors for the first time, and is of course included in the present invention. A typical chimera antibody includes a heavy chain and/or a light chain variable region (including CDR and FR) of the immunoglobulin of one type of animal, typically mouse, fused with the constant region of another animal, typically man, and in the present invention those formed by fusing the variable region of an anti-prion antibody produced by one described in the above-mentioned publications, hybridoma clone 72-5, 44B1, etc. with the constant region of a human antibody, etc. can be cited as examples.

One embodiment of the anti-prion chimera antibody of the present invention includes one that is coded for by
an antibody heavy chain gene selected from the group consisting of
(1a) a nucleic acid that contains a sequence selected from the group consisting of SEQ ID NOS: 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for, and
   an antibody light chain gene selected from the group consisting of
(2a) a nucleic acid containing a sequence selected from the group consisting of SEQ ID NOS: 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for.
The primatized and humanized antibody typically includes a heavy chain and/or light chain CDR from a mouse antibody grafted onto a primate or human antibody variable region framework, and normally further includes a human constant region.

Furthermore, it is also possible to obtain an antibody useful in the present invention by freshly producing an anti-prion antibody or a fragment thereof and screening it with respect to PrP^{sc} growth inhibitory activity. Although prion protein has very weak antigenicity and it has been difficult to prepare an antibody by a standard immunization method, due to the development by Bueler et al. of a prion gene knockout mouse (Prnp-/- mouse) (Bueler H et al. 1992. Nature 356(6370): 577-82) it has become possible to prepare an anti-prion antibody by immunization of the mouse with an antigen containing a prion protein or an epitope thereof in accordance with a standard method. In the present invention, it should be noted that an anti-prion antibody prepared by a method other than one employing the Prnp-/mouse is not excluded. Examples of the antigen include a homogenate of PrP^{Sc} infected tissue, a purified product thereof, or a modified product thereof, a synthetic peptide containing an amino acid sequence of part of the prion protein, and a recombinant prion protein, but are not limited thereby.

Cloning of an anti-prion antibody gene may be carried out by any technique that is well known in the art. For example, a desired antibody gene may be obtained by constructing a cDNA library based on RNA of an anti-prion antibody-producing cell and using a antibody-specific primer. More specifically, a polynucleotide containing a nucleic acid sequence of an unknown variable region may be obtained by, for example, preparing an antibody constant region-specific primer and employing a known technique such as a RACE (Rapid Amplification of cDNA End) method, but the anti-prion antibody gene of the present invention is not limited to one obtained by such a method. The polynucleotide thus obtained may be digested by a predetermined restriction enzyme and cloned to any known vector.

Examples of the anti-prion antibody gene that can be used in the present invention include one comprising an antibody heavy chain gene and an antibody light chain gene, in which the antibody heavy chain gene is selected from the group consisting of
(1a) a nucleic acid that contains a sequence selected from the group consisting of SEQ ID NOS: 1, 3, 5, 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for, and
   the antibody light chain gene is selected from the group consisting of
(2a) a nucleic acid containing a sequence selected from the group consisting of SEQ ID NOS: 2, 4, 6, 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for,
   the antibody obtained by expression of the antibody heavy chain gene and the antibody light chain gene having PrP^{Sc} growth inhibitory activity.
Among these genes, those having a higher PrP^{Sc} growth inhibitory activity when expressed are preferable in the present invention.

The term 'stringent conditions' used in the present specification refers to parameters that are well known in the art. Parameters for the hybridization of a nucleic acid are described in standard protocols such as, for example, Sambrook et al., Molecular Cloning: A Laboratory Manual, 3d ed., Cold Spring Harbor Press (2001), or Ausubel et al., Current Protocols in Molecular Biology, Greene Publishing Associates (1992).

Specifically, the stringent conditions used in the present specification mean hybridization at 65°C by means of a hybridization buffer containing 3.5× SSC, Ficol 0.02%, polyvinylpyrrolidone 0.02%, bovine serum albumin 0.02%, NaH₂PO₄ 25 mM (pH7), SDS 0.05%, and EDTA 2 mM. Among the above-mentioned components, SSC is 0.15 M sodium chloride /0.15 M sodium citrate at pH 7, SDS is sodium dodecylsulfate, and EDTA is ethylenediaminetetraacetic acid. After the hybridization, a membrane to which DNA is transferred is washed with 2× SSC at room temperature, then with 0.1 to 0.5× SSC/0.1× SDS at a temperature up to 68°C. Alternatively, the stringent hybridization may employ hybridization and washing conditions described by a manufacturer using a commercial hybridization buffer such as an ExpressHyb (registered trademark) buffer solution (manufactured by Clontech Corp.).

There are other conditions, reagents, etc. that can be used and give the same degree of stringency, but since it can be expected that a person skilled in the art knows such conditions very well, there is no particular description thereof in the present specification. However, it is possible to manipulate the conditions so that a homologue or an allelic gene of a nucleic acid that codes for the mutant of the present invention can be clearly identified.

When introducing a gene that imparts PrP^{Sc} growth inhibitory activity to mesenchymal cells, various known methods may be used. For example, the gene can be introduced into mesenchymal cells by a method in which the gene is integrated into a virus vector, and this vector is introduced into the mesenchymal cells by infection, a calcium phosphate transfection method (Berman et al. 1984. Proc. Natl. Acad. Sci. USA 81: 7176), or many well-known techniques involving direct delivery of a gene employing DEAE-dextran transfection, protoplast fusion (Deans et al. 1984. Proc. Natl. Acad. Sci. USA 81: 1292), electroporation, liposome fusion, transfection by means of polybrene, and laser microporation of a cell membrane. Moreover, a person skilled in the art may apply to the present invention any technique other than the above-mentioned techniques that enables the gene to be integrated into the genome of a cell and to be appropriately introduced into the cell so that the gene can be expressed.

When used in the present specification, the 'vector' means any nucleic acid that is capable of introducing a desired nucleic acid molecule by digestion or ligation in order to transfer between different genetic environments or in order to carry out expression in a host cell. The vector is typically constituted from DNA, but an RNA vector may also be used. The vector includes a plasmid, a phagemid, and a virus genome, but should not be limited thereto. A cloning vector can replicate in a host cell autonomously or after integration into a genome and is further characterized by one or more endonuclease restriction sites, the vector is cleaved in a decidable manner at these sites, a desired nucleic acid sequence can be linked thereto, and a novel recombinant vector can thereby replicate a target nucleic acid molecule in a host. In the case of a plasmid, by increasing the plasmid copy number in the host bacterium, a desired nucleic acid molecule may be replicated any number of times, or replication may be carried out only once per host before the host is regenerated by cell division. In the case of a phage, replication may occur actively between lytic phases, or may occur passively between lysogenic phases.

With regard to an expression vector, a desired nucleic acid sequence is inserted thereinto by digestion and ligation, operably linked to a regulatory sequence, and expressed as a transcript.
The gene used in the present invention may be constituted from one or more genes, and when it is constituted from two or more genes, these genes may be inserted into a single expression vector or may be inserted separately into two or more vectors.
The expression vector may further contain one or more marker sequences that are suitable for identifying a cell that is or is not transformed or transfected by the vector. The marker contains, for example, a gene that codes for a protein that increases or decreases either the resistance or the sensitivity toward an antibiotic or another compound, a gene that codes for an enzyme (e.g. β-galactosidase, luciferase, or alkaline phosphatase) whose activity is detectable by a standard analytical method in the art, and a gene that visually affects the phenotype of a transformed or transfected cell, a host, a colony, or a plaque. A preferred expression vector is a vector that enables autonomous replication and expression of a structural gene product present in a DNA segment to which the vector is operably linked.

In the present specification, the code sequence and the regulatory sequence are said to be 'operably' linked when they are linked in a manner in which expression or transcription of the code sequence is under the influence or control of the regulatory sequence. If it is desired that the code sequence is translated into a functional protein, the two DNA sequences are said to be 'operably' linked if, as a result of induction by a promoter in a 5' regulatory sequence, transcription of the code sequence occurs, or if linkage properties between the two DNA sequences (1) do not result in induction of frameshift mutation, (2) do not interfere in the ability of the promoter for instructing transcription of the code sequence, or (3) do not interfere in the ability of a corresponding RNA transcript to be translated into a protein. Therefore, the promoter region is operably linked to the code sequence if the promoter region can transcribe the DNA sequence so that the resulting transcript is translated into a desired protein or polypeptide.

A useful vector in the present invention contains a nucleic acid molecule that codes for an antibody of the present invention that functionally binds to an appropriate transcriptional or translational regulatory sequence that is derived from a gene of, for example, a mammal, a microbe, a virus, or an insect as desired. Such a regulatory sequence includes a sequence having a regulatory role in gene expression such as, for example, a transcription promoter or enhancer, an operator sequence for regulating transcription, a sequence that codes for a ribosome-binding site within a messenger RNA, and an appropriate sequence that regulates transcription, translation initiation, or transcription termination.

Detailed properties of the regulatory sequence necessary for gene expression may be different depending on the species of organism or species of cell, but it can generally contain at least 5' nontranscribed and 5' untranslated sequences involved in initiation of transcription and translation, such as a TATA box, a capping sequence, and a CAAT sequence. In particular, such a 5' nontranscribed regulatory sequence can contain a promoter region that contains a promoter sequence for regulating the transcription of an operably linked gene. The regulatory sequence also contains an enhancer sequence or a desired upstream activator sequence. The vector of the present invention may optionally contain a 5' leader or signal sequence. Selection and design of an appropriate vector are within the ability and freedom of a person skilled in the art.

A particularly useful regulatory sequence contains a promoter region derived from a gene of various mammals, viruses, microbes, and insects. This promoter region commands initiation of the transcription of a target gene, thus resulting in transcription of the whole DNA containing the anti-prion antibody gene. A useful promoter region includes a CAG promoter, a retroviral dLTR promoter, a cytomegalovirus (CMV) enhancer / promoter region, an RSV LTR promoter, a lac promoter, and a promoter separated from an adenovirus, but any other promoters known to a person skilled in the art that are useful for gene expression in a eucaryote, a procaryote, a virus, or a microbial cell may be used.

Other particularly useful promoters for expressing a gene or a protein within a eucaryote cell include mammalian cell promoter and enhancer sequences such as, for example, those induced from polyomavirus, adenovirus, SV40 virus, and human cytomegalovirus. Typically, virus early and late promoters, which are found adjacent to the virus replication origin of a virus such as SV40, are particularly useful. Selection of a specific useful promoter depends on various other parameters pertaining to the cell line and the nucleic acid construct used for expressing the antibody of the present invention within a specific cell line. Furthermore, any promoter that is known to express a gene in a target cell at a sufficiently high level to be useful in the present invention may be selected.

The above-mentioned expression vector may further contain, as desired, various regulatory sequences that include a nucleic acid sequence that codes for various signal peptides that may functionally bind to a gene that imparts PrP^{Sc} growth inhibitory activity and a ribosome binding site that enables efficient translation into a protein of mRNA generated from the expression vector. The signal peptide, if present, is expressed as a precursor amino acid that enables the extracellular secretion of a translated polypeptide to be improved.
The nucleic acid construct of the present invention therefore includes various forms of the nucleic acid molecule of the present invention that operably binds to either of the promoter sequence or the promoter and enhancer sequence, and that further functionally binds to a polyadenylation sequence that commands termination and polyadenylation of mRNA. The nucleic acid construct of the present invention can contain another gene sequence that enables efficient replication and expression of the construct within a desired cell. Such a sequence can include an intron derived from a viral gene, etc.

Examples of the virus vector that can be suitably used for introduction of a gene into mesenchymal cells in the present invention include a modified adenovirus described in JP, A, 2002-330789. The adenovirus has a modified fiber protein obtained by joining a molecule having specificity for a specific type of cell and/or tissue to a fiber protein that has substantially no ability to bind to a CAR (Coxsackie adenovirus receptor), which is a main receptor for a wild-type adenovirus, and/or a modified fiber protein obtained by modifying, so as to have specificity for a specific type of cell and/or tissue, a fiber protein that has substantially no ability to bind to a CAR, and in the present invention it is particularly preferable to employ one having a modified fiber protein obtained by joining a molecule containing an RGD (Arg-Gly-Asp) motif to a fiber protein that has substantially no ability to bind to a CAR.

Such a modified adenovirus vector may be produced by a well-known molecular biological technique in the art such as, for example, a method that uses a genome - terminal protein complex (hereinafter, abbreviated to DNA-TPC), which keeps a terminal protein covalently bonded to opposite ends of the virus genome (ref. Yoshida et al. 1998. Hum Gene Ther 9: 2503-2515, etc.). Such a method is well known to a person skilled in the art. Typically, while producing a cosmid in which a target gene is inserted into a cosmid cassette such as pAxCw or pAxCAwt, DNA-TPC is produced from a virus and cleaved by an appropriate restriction enzyme. Subsequently, the above-mentioned cosmid and the DNA-TPC treated with the restriction enzyme are cotransfected to an appropriate host cell such as, for example, a 293 cell. Following this, culturing is carried out under appropriate conditions for a predetermined period, and recombinant adenovirus particles released as virus particles into the culture liquid may be collected.

It is possible to introduce into the mesenchymal cells used in the present invention, in addition to the above, any gene that has an effect in enhancing the therapeutic effect on a prion disease such as, for example, a gene that promotes cell growth or differentiation into neurons, a gene that increases cell survival rate, a gene that increases the lifespan of a cell (e.g. a telomerase gene: see WO 03/038075), a gene that regulates the cell cycle, a gene that improves the migratory capacity of a cell, a gene that has neuroprotective action, or a gene that has an apoptosis inhibiting effect.
The above-mentioned modification carried out with respect to mesenchymal cells may be carried out for any other types of cells as appropriate, and modified cells thus produced may be used for inhibiting the growth of an abnormal prion. Such modified cells are included in the present invention.

The remedy for a prion disease of the present invention may be administered as an agent (composition) to which various substances having an effect in enhancing the therapeutic effect on a prion disease are added. As examples of such substances, for example, those having PrP^{Sc} growth inhibitory activity can be cited. Examples of PrP^{Sc} growth inhibitor substances include compounds like amphotericin B, Congo red, anthracycline, cysteine protease inhibitors (Doh-Ura K et al. supra) such as quinacrine, tilorone, chloroquine, and E-64d, phenothiazine derivatives (Korth C et al. supra) such as promazine, chloropromazine, and acepromazine, tetrapyrroles (Caughey WS et al. supra) such as porphyrin and phthalocyanine, pentosan polysulfate, reactive dyes such as reactive green and reactive red, and quinines (JP, A, 2003-40778) such as quinine, 8-hydroxyquinoline-2-carboxaldehyde 8-quinolylhydrazone, 2-pyridinecarboxaldehyde 2-quinolylhydrazone, and 2,2'-biquinoline, gene constructs that code for the PrP^{c} dominant negative mutant (Non-patent Publication 1), and substances that bind to PrP^{c} and/or PrP^{Sc}, but are not limited thereto.

Examples of substances that bind to PrP^{c} and/or PrP^{Sc} include an anti-prion antibody or a fragment thereof (e.g. Non-patent Publication 2, Non-patent Publication 3, JP, A, 2003-144148, JP, A, 2003-321498, Enari M et al. supra, Peretz D et al. supra, Gilch S et al. supra), a cadherin such as protocadherin 43 or OB-cadherin-1 (JP, A, 2000-51213), and a plasminogen, a plasminogen fragment, and a derivative thereof (JP, A, 2004-501626), and among them those having PrP^{Sc} growth inhibitory activity are preferable.

Furthermore, it can be determined whether or not a given substance has PrP^{Sc} growth inhibitory activity by a well known method described in JP, A, 2003-149237, etc., and any PrP^{Sc} growth inhibitor identified by such a method (e.g. chlorophyll a, chlorophyll b, copper chlorophyll, sodium copper chlorophyllin, sodium iron chlorophyllin) may be used in the agent of the present invention.
Examples of substances that have an effect in enhancing the therapeutic effect on a prion disease include, in addition to the above, a substance that promotes cell growth or differentiation into neurons, a substance that increases cell survival rate, a substance that increases the lifespan of a cell, a substance that regulates the cell cycle, a substance that improves the migratory capacity of a cell, a substance that has a neuroprotective action, and a substance that has an apoptosis-inhibiting effect.

When producing the remedy of the present invention, the mesenchymal cells (including modified ones) produced as above may be used as they are, but after the cells are produced and, as necessary, grown, they may be cryopreserved, and thawed and used for production of the remedy as required.
The prion remedy of the present invention may be made into a preparation by a method known to a person skilled in the art. For example, it may be used parenterally in an injectable form of an aseptic solution or a suspension liquid agent with water or another pharmaceutically acceptable liquid as necessary. For example, it can be expected that a preparation may be made by mixing a generally recognized and pharmaceutically required unit dose thereof with an appropriate combination of a pharmacologically acceptable carrier or medium, specifically, sterile water, a saline solution, a vegetable oil, an emulsifier, a suspending agent, a surfactant, a stabilizer, a diluent, a vehicle, an antiseptic, or a binder, etc. The amount of effective component in these preparations is adjusted so as to obtain an appropriate dosage in the prescribed range. Furthermore, an aseptic composition for injection may be formulated in accordance with a normal preparation method using a vehicle such as distilled water for injection.

Examples of an aqueous solution for injection include a saline solution, an isotonic solution containing glucose or another adjuvant, such as, for example, D-sorbitol, D-mannose, D-mannitol, or sodium chloride, and an appropriate dissolution adjuvant such as, for example, an alcohol, specifically ethanol, a polyalcohol, specifically, propylene glycol or polyethylene glycol, a nonionic surfactant such as, for example, polysorbate 80, HCO-50, etc. may be used in combination.
Examples of oily liquids include sesame oil and soya oil, and as a dissolution adjuvant benzyl benzoate, benzyl alcohol, etc. may be used in combination. Furthermore, a buffer such as a phosphate buffer or a sodium acetate buffer, a soothing agent such as procaine hydrochloride, a stabilizer such as benzyl alcohol or phenol, an antioxidant, etc. may be added. The injection solution thus prepared is usually charged into an appropriate container such as an ampoule, a vial, a tube, a bottle, or a pack.

Administration into a subject's body may employ any route, but parenteral administration is preferable, and local administration or intravenous administration is particularly preferable. The number of doses administered is preferably one, but a plurality of doses may be administered according to the circumstances. Furthermore, the administration time may be a short period of time or it may be continuous administration over a long period of time. More specifically, the remedy of the present invention may be administered by injection or percutaneously. Examples of the administration by injection include intravenous injection, intraarterial injection, selective intraarterial injection, intramuscular injection, intraperitoneal injection, subcutaneous injection, intraventricular injection, intracerebral injection, and injection into cerebrospinal fluid space, but are not limited thereto.
In the case of intravenous injection, administration is possible by a normal procedure for blood transfusion, there is no need for surgery on the subject, there is no need for local anesthesia, and it is therefore possible to alleviate the burden on both the subject and a technician. Moreover, it is advantageous since administration is possible outside an operating theater.
The anti-prion chimera antibody and the vector of the present invention may be formulated and administered by the same methods as above.

Furthermore, the present invention relates to a method of treating a prion disease, the method comprising administering to a subject a therapeutically effective amount of the remedy, the vector, the anti-prion chimera antibody, and/or a sustained-release preparation, which will be described in detail later, of the present invention.
Mesenchymal cells such as bone-marrow cells, umbilical cord blood cells, or peripheral blood cells contained in the agent used for the above-mentioned treatment method are preferably, unless a special operation such as immunosuppression is carried out, those harvested from the subject's own body or those derived from the above (subject-derived autologous cells) (autotransplantation therapy) in order to prevent the possibility of a rejection reaction due to administration. Such an embodiment is preferable since the combined use of an immunosuppressant can be avoided. Although it is possible to use allogeneic cells by carrying out an immunosuppressive treatment, overwhelmingly better therapeutic effects can be expected by the use of autologous cells.

When it is difficult to use autologous cells, it is possible to use cells derived from another subject or another animal for medical use. Cryopreserved cells may be used.
The above-mentioned autologous cells may be any of those harvested from the body of the subject in an undifferentiated state, those formed by subjecting mesenchymal stem cells harvested from the body of the subject in an undifferentiated state to genetic engineering, and those formed by carrying out differentiation-induction on mesenchymal stem cells harvested from the body of the subject in an undifferentiated state.
Furthermore, in the treatment method of the present invention, administration of the agent of the present invention (containing mesenchymal cells, for example, bone-marrow cells) to a subject may be carried out desirably by, for example, the above-mentioned method. Moreover, a physician or a veterinarian may alter the above-mentioned method as appropriate for administering the agent of the present invention to a subject.
Furthermore, the subject of the above-mentioned treatment method of the present invention is not necessarily limited to man. Normally, the method of the present invention may be carried out in the same manner using mesenchymal cells in a mammal other than man (e.g. a rodent such as a mouse, a rat, a sand rat, or a guinea pig, an animal of the feline family such as a cat, a puma, or a tiger, a cervid such as a deer or an elk, a mink, a sheep, a goat, a cow, a monkey, etc.).

Another aspect of the present invention relates to a method of treating a prion disease, the method comprising sustainedly releasing a therapeutically effective amount of an anti-prion antibody, and a sustained-release preparation used in such a treatment method.
Sustained release may be achieved by the use of any sustained-release preparation, and is typically carried out in the body of a subject, but is not limited thereto, and it may be carried out via a sustained-release preparation or sustained-release device placed outside the body or on the surface of the body. Examples of the sustained-release preparation include an osmotic pump and a molding such as, for example, a semipermeable polymer matrix in the form of a film or microcapsules. As the osmotic pump, for example, an Alzet (registered trademark) osmotic pump manufactured by DURECT, etc. may be suitably used. Since this pump can release a solution contained therewithin in a predetermined flow pattern, by charging a pump having desired release characteristics with a solution of an anti-prion antibody in a physiologically acceptable solvent such as saline solution or PBS and placing this within the body, sustained release of the antibody can easily be achieved. Examples of a sustained-release matrix include polylactide (US Pat. No. 3,773,919 and EP Pat. No. 58481), a copolymer of L-glutamic acid and γ-ethyl-L-glutamate (Sidman, U. et al., Biopolymers 22: 547-556 (1983)), poly(2-hydroxyethyl methacrylate) (R. Langer et al., J. Biomed. Mater. Res. 15: 167-277 (1981), and R. Langer, Chem. Tech. 12: 98-105 (1982)), ethylene vinylacetate (R. Langer et al. supra), and poly-D-(-)-3-hydroxybutyric acid (JP, A, 60-54326). The sustained-release preparation also includes a liposome-entrapped polypeptide. Liposome containing an anti-prion antibody is produced by a known method (ref. e.g. Epstein et al. 1985. Proc Natl Acad Sci USA 82: 3688-3692, Hwang et al. 1980. Proc Natl Acad Sci USA 77: 4030-4034, etc.). The liposome is normally of a small (about 200 to 800 Å) single layer type, the lipid content thereof is higher than about 30 mol% cholesterol, and the selectivity ratio is adjusted according to an optimum antibody treatment.

The sustained-release preparation of the present invention may contain cells that secrete an anti-prion antibody. Examples of such cells include not only an anti-prion antibody-producing hybridoma such as clone 44B1 or 72-5 but also any cells into which an anti-prion antibody gene is introduced. Since the sustained-release preparation containing such cells can release an antibody as long as the cells survive, it becomes possible to advantageously decrease the frequency of introduction of the preparation, which may involve an invasive method. Among such cells, it is preferable to use self-derived cells, which have little effect on the living body, and it is also preferable to use cells for which the growth or the life and death thereof can be controlled. In the sustained-release preparation of the present invention, mesenchymal cells into which an anti-prion antibody gene has been introduced are particularly preferable, and mesenchymal stem cells are more preferable, and since by administering such cells not only can the anti-prion antibody be sustainedly released, but also the cells reconstruct damaged nerve tissue, a synergistic effect can be expected. The above-mentioned cells may contain any gene that has an effect in enhancing the therapeutic effect on a prion disease such as, for example, a gene that promotes cell growth or differentiation into neurons, a gene that increases cell survival rate, a gene that increases the lifespan of a cell (e.g. a telomerase gene), a gene that regulates the cell cycle, a gene that improves the migratory capacity of a cell, a gene that has neuroprotective action, or a gene that has an apoptosis inhibiting effect.
The above-mentioned preparation containing anti-prion antibody-secreting cells may be provided as a suspension containing the cells in a physiologically acceptable solvent such as saline solution, PBS, or various cell culture media, or a capsule agent containing the suspension in a biodegradable capsule. Furthermore, the above-mentioned cells may be cryopreserved and used by thawing as necessary. Other examples of making a preparation are as already described for the remedy of the present invention, and they are well known to a person skilled in the art.

The sustained-release preparation of the present invention may be introduced into any part of the body such as, for example, subcutaneous tissue, muscle, abdominal cavity, brain, cerebral ventricle, cerebrospinal fluid space, artery, vein, etc. by a standard method. The release period is not particularly limited as long as the progress of a prion disease is delayed or the clinical state is improved, and good therapeutic effects can be obtained by a period of, for example, 10 to 50 days. The amount released is not particularly limited as long as the progress of a prion disease is delayed or the clinical state is improved, but it is typically 0.01 to 100 mg/kg/day, preferably 0.1 to 10 mg/kg/day, and more preferably 0.5 to 5 mg/kg/day. When a preparation containing anti-prion antibody-secreting cells is used, although it depends on the ability of the cells to produce antibody, the dosage is typically 1 × 10⁴ to 1 × 10⁷ cells/kg (local administration) or 1 × 10⁶ to 1 × 10⁹ cells/kg (intravenous administration), preferably 1 × 10⁵ to 1 × 10⁶ cells/kg (local administration) or 1 × 10⁷ to 1 × 10⁸ cells/kg (intravenous administration), and more preferably 5 × 10⁵ to 1 × 10⁶ cells/kg (local administration) or 5 × 10⁷ to 1 × 10⁸ cells/kg (intravenous administration). Such a dosage can be adjusted as appropriate according to various conditions of the treatment such as, for example, the severity of the disease, the general health state of the subject, age, weight, sex, diet, administration route of the preparation, administration timing and administration frequency, whether or not there is concomitant use of a medicine, sensitivity toward a reaction, and tolerance and response to the treatment.

The remedial method of this aspect of the present invention includes introducing an anti-prion antibody gene into cells of a subject. Since cells into which an anti-prion antibody gene has been introduced sustainedly release the anti-prion antibody, they can act as the sustained-release preparation of the present invention. A method of introducing a nucleic acid molecule into desired cells of a subject is well known, and this includes the use of a vector and injection of various nucleic acid constructs into a subject. The cells into which it is introduced are not particularly limited as long as an effect in improving a prion disease can be obtained, and typical examples thereof include cells present in the brain of a subject.
Large numbers of various types of vectors that can appropriately deliver a gene and express a desired nucleic acid molecule have been developed, and are described in publications such as, for example, Current Comm. Mol. Biol., Cold Springs Harbor Laboratory, New York (1987). As vectors containing an anti-prion antibody gene, in addition to those described in the publication, the above-mentioned various types may suitably be used. When a vector is used, typically, a therapeutically effective amount of the vector containing the nucleic acid molecule of the present invention is introduced into a circulation system or locally into a subject so that the vector can specifically infect desired cells. In another preferred embodiment, the vector is directly injected into the brain of a subject. The dosage of the vector is not particularly limited as long as the prion disease is improved and, for example, the adenovirus vector of the present invention may be administered at 10³ to 10¹⁵ viral particles per adult. Such a dosage can be adjusted as appropriate according to various conditions of the treatment such as, for example, the severity of the disease, the general health state of the subject, age, weight, sex, diet, administration route of the preparation, administration timing and administration frequency, whether or not there is concomitant use of a medicine, sensitivity toward a reaction, and tolerance and response to the treatment.
The present invention further aims to directly inject into a subject a nucleic acid construct having a promoter, an anti-prion antibody gene, and its subsequent polyadenylation sequence. A useful example of such a method is described in Vile et al. 1994. Ann Oncol 5 Suppl 4: 59. Such a nucleic acid construct may be injected into the muscle or another part of a subject or directly into the brain of a subject.

In accordance with another aspect of the present invention, there is provided the use of mesenchymal cells in the production of an agent for delivering a substance to a lesion site of a prion disease, and an agent that contains mesenchymal cells and delivers a substance to a lesion site of a prion disease. The substance referred to here is not particularly limited as long as it can be inserted into mesenchymal cells or attached to the surface of a cell, and examples thereof include a substance that has an effect in enhancing the therapeutic effect on a prion disease such as a substance that inhibits the growth of an abnormal prion, a substance that promotes cell growth or differentiation into neurons, a substance that increases cell survival rate, a substance that increases the lifespan of a cell, a substance that regulates the cell cycle, a substance that improves the cell migratory capacity, a substance that has a neuroprotective action, and a substance that has an apoptosis inhibiting effect and, furthermore, a pigment, an enzyme, a labeled substance such as a fluorescent substance or a radioactive isotope, and a vector for introducing any gene into surrounding cells. Among such substances, those useful for research, diagnosis, treatment, etc. of a prion disease are preferable.
The above-mentioned delivery agent may be produced by the same method as the above-mentioned method for the remedy of the present invention, and administered to a subject.

### EXAMPLES

The present invention is explained in detail by reference to Examples below, but the scope of the present invention is not limited by these Examples.

### Example 1: Production of anti-PrP monoclonal antibody-producing hybridoma

A prion protein knockout mouse (Prnp-/- mouse) was immunized with, as an antigen, purified recombinant PrP (rPrP) or purified abnormal prion protein (PrP^{Sc}) from the brain of a scrapie Obihiro strain-infected mouse, spleen cells thus obtained and a myeloma cell line (P3U1) were fused to give a hybridoma, and this was screened with respect to its ability to bind to PrP (ref. Kim et al. 2004. Virology 320: 40-51).

### (1) Antigen preparation

E. Coli was made to express a recombinant polypeptide (rPrP) corresponding to positions 23 to 231 of mouse PrP using E. Coli expression vector pRSETB (manufactured by Invitrogen). Since the rPrP was expressed in an inclusion body, this was solubilized by 8 M guanidine hydrochloride and purified with Ni2+-IMAC. The purified product was subjected to reverse-phase HPLC with respect to one having an SS bond within the molecule to give further purified rPrP as an antigen (ref. Kim et al. supra).

### (2) Immunization of mouse

As a subject animal, a 10 to 12 week old Prnp-/mouse (Yokoyama et al. 2001. J Biol Chem 276: 11265-11271) was used. The mouse was subcutaneously inoculated with 200 µg of the above-mentioned antigen together with Freund's Complete Adjuvant. Following this, subcutaneous inoculation with 100 µg of the above-mentioned antigen together with Freund's Incomplete Adjuvant was carried out every two weeks. A final immunization was carried out by injecting 50 µg of the above-mentioned antigen via a tail vein.

### (3) Cell fusion and selection of hybridoma

On the 42nd day after the first immunization, the spleen was extracted from the immunized mouse under deep anesthesia, and a single cell suspension was prepared. The cells thus obtained were fused with P3U1 cells using polyethylene glycol 1500. The culture supernatant of the hybridoma thus obtained was screened by ELISA using rPrP and purified PrP^{Sc} as antigens. A hybridoma that produced an antibody that reacted with rPrP or purified PrP^{sc} was cloned by a limiting dilution method.

### Example 2: PrP^{sc} growth inhibitory activity of anti-PrP monoclonal antibody

Among antibodies obtained in Example 1, one having PrP^{sc} growth inhibitory activity was selected. 13/15 cells with a persistent prion infection (Race et al. 1987 J Gen Virol 68: 1391-9) were cultured in a medium containing a concentration of 0.1 to 10 µg/mL of anti-PrP monoclonal antibody for 3 days. After the medium was removed, the cells were washed with PBS, dissolved in a buffer solution containing 0.5% Triton-X100 and 0.5% sodium deoxycholate, and digested with 20 µg/mL of protein kinase K at 37°C for 20 minutes. After the activity of the protein kinase K was terminated by adding 2 mM of Pefablock, PrP^{Sc} was collected by centrifugation at 100,000× g for 2 hours. PrP^{Sc} was detected by the Western blot method.

Among Examples below, three types of hybridomas, that is, 43C5 (IgG1), which did not show PrP^{Sc} growth inhibitory activity, and 72-5 (IgG1, Depository No.: FERM P-18516) and 44B1 (IgG₂ₐ, Depository No.: FERM P-18515), which showed PrP^{Sc} growth inhibitory activity, were used.

### Example 3: Identification of anti-prion antibody gene

### (1) Cloning of antibody gene

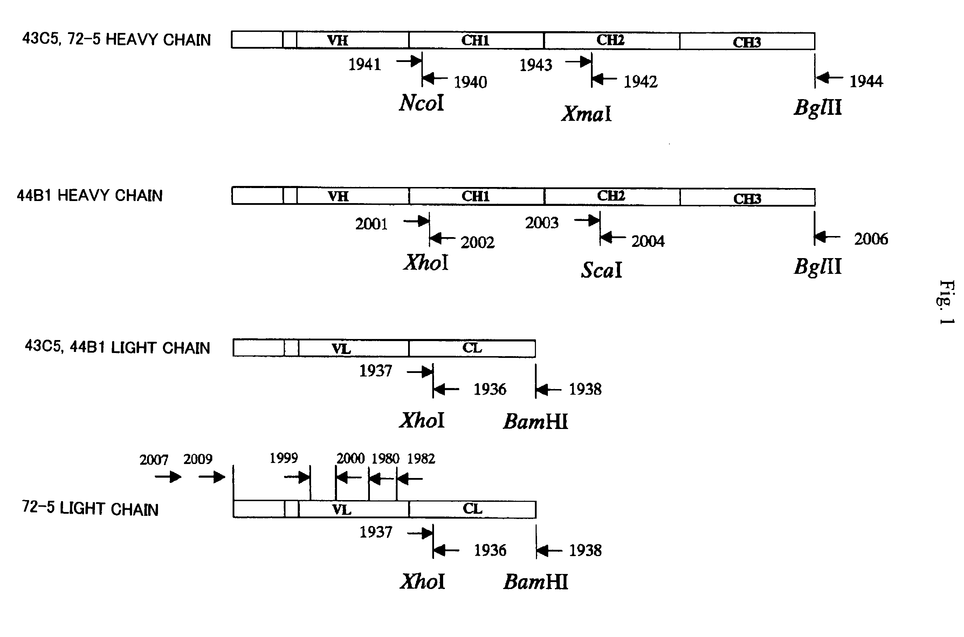

Total RNA was prepared from the hybridoma selected in Example 2 in accordance with a standard method, and antibody genes (heavy chain, light chain) were cloned by a 5'-RACE (Rapid Amplification of cDNA End) method and an RT-PCR method respectively. The 5'-RACE method was carried out using a SMART (registered trademark) RACE cDNA Amplification Kit (manufactured by CLONTECH). The primers shown below were prepared for cloning antibody genes. FIG. 1 shows the correlation between antibody genes and primers.

### [Table 1]

**Table 1 List of primers used for cloning of anti-PrP antibody genes**

| | |
|---|---|
| 1936 (SEQ ID NO: 9) : | 35mer 5'- TCACTCGAGGGTGGGAAGATGGATACAGTTGGTGCA |
| 1937 (SEQ ID NO:10) : | 32mer 5'- ACCCTCGAGTGAGCAGTTAACATCTGGAGGTG -3' |
| 1938 (SEQ ID NO:11) : | 36mer 5'- CGCGGATCCTTAACACTCATTCCTGTTGAAGCTCTT- |
| 1940 (SEQ ID NO:12) : | 23mer 5'- CTTGACCAGGCATCCCAGGGTCA -3' |
| 1941 (SEQ ID NO:13) : | 25mer 5'- CCTGGATCTGCTGCCCAAACTAACT -3' |
| 1942 (SEQ ID NO:14) : | 26mer 5'- CGGAAAGTGCTGTTGAACTGCTCCT -3' |
| 1943 (SEQ ID NO:15) : | 24mer 5'-AGGTGCACACAGCTCAGACGCAAC -3' |
| 1944 (SEQ ID NO:16) : | 33mer 5'- CCGAGATCTCATTTACCAGGAGAGTGGGAGAGG -3' |
| 1980 (SEQ ID NO:17) : | 26mer 5'- CGGAGATTTTGCATATTGCAGCAGT -3' |
| 1982 (SEQ ID NO:18) : | 28mer 5'- AATAGTCTACAAAATCTTCAGACTCAAG -3' |
| 1999 (SEQ ID NO:19) : | 23mer 5'- CAGTCTCACTTGTCGGGCAAGTC -3' |
| 2000 (SEQ ID NO:20) : | 26mer 5'- ATCTAAACTGGATGTGCCGTAGATCA -3' |
| 2001 (SEQ ID NO:21) : | 23mer 5'- GAGCCAGTGACCTTGACCTGGAA-3' |
| 2002 (SEQ ID NO:22) : | 23mer 5'- CACATTGCAGGTGATGGACTGGC -3' |
| 2003 (SEQ ID NO:23) : | 25mer 5'- AGTACACAGCTCAGACACAAACC -3' |
| 2004 (SEQ ID NO:24) : | 23mer 5'- CTCATCCAGTCCTGGTGCTGGAT -3' |
| 2006 (SEQ ID NO:25) : | 24mer 6'- CCGAGATCTCATTTACCCGGAGTC -3' |
| 2007 (SEQ ID NO:26) : | 26mer 5'- ATTGTCATTGCAGTCAGGACTCAGCA -3' |
| 2009 (SEQ ID NO:27) : | 24mer 5'- ACTCAGCATGGACATGAGGGCTCC -3' |

RACE cDNA was prepared as follows. That is, 1 µg of total RNA of 43C5, 72-5, and 44B1, 1 µL of 5'-CDS primer, and 1 µL of SMART II A (registered trademark) oligonucleotide (SEQ ID NO: 7) were mixed with deionized water to make 5 µL, and the mixture was heated at 70°C for 2 minutes, and rapidly cooled in ice for 2 minutes. Subsequently, 2 µL of 5x First-Strand buffer, 1 µL of DTT (20 mM), 1 µL of dNTP mix (10 mM), and 1 µL of PowerScript (registered trademark) reverse transcription enzyme were added thereto to make the total amount 10 µL, and the mixture was incubated at 42°C for 90 minutes. Subsequently, the mixture was heated at 72°C for 7 minutes to give 5'-RACE cDNA. The 5'-CDS primer shown in Table 2 below was used.

### [Table 2]

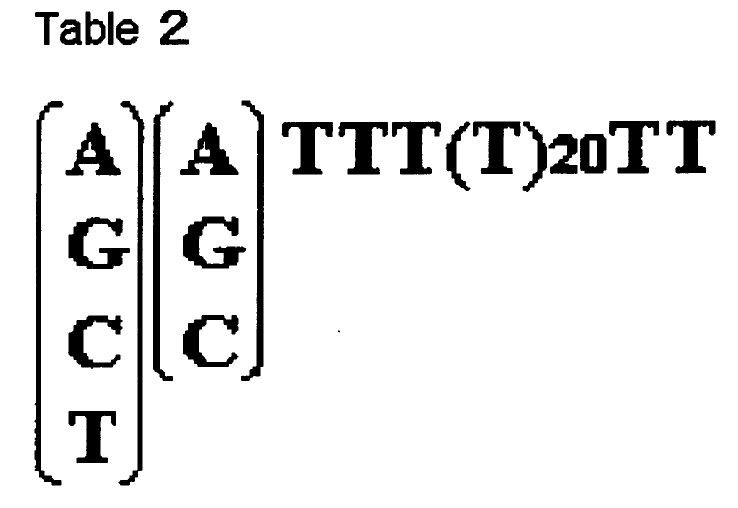

A 5'-RACE of an antibody variable region was prepared in accordance with a Clontech manual using a master mix (Table 4) and a RACE reaction solution (Table 5) having the composition below. As a universal primer, a mixture formed from the following combination (UPM: Universal Primer A Mix) was used.

### [Table 3]

**Table 3**

| | |
|---|---|
| Long (0. 4 mM): | 5' - CTAATACGACTCACTATAGGGCAAGCAGTGGTATCAACGCAGAGT - 3' (SEQ ID NO: 8) |
| Short (2 mM): | 5' - CTAATACGACTCACTATAGGGC - 3' |

As the 5'-RACE primer (GSP: Gene Specific Primer), 1940 (43C5, 72-5 heavy chain), 2002 (44B1 heavy chain), and 1936 (43C5, 44B1 light chain) were used. A reaction was carried out under conditions of 5 cycles of 94°C 5 seconds and 72°C 3 minutes; 5 cycles of 94°C 5 seconds, 70°C 10 seconds, and 72°C 3 minutes; and 25 cycles of 68°C 10 seconds and 72°C 3 minutes.

### [Table 4]

**Table 4 Composition of master mix**

| | |
|---|---|
| PCR grade water | 30.5 µL |
| 10x Advantage-HF 2 PCR buffer | 5 µL |
| 10x Advantage-HF 2 dNTP mix | 5 µL |
| 50x Advantage-HF 2 polymerase mix | 1 µL |
| Total | 41.5 µL |

### [Table 5]

**Table 5 Composition of 5'-RACE reaction mixture (units: µl)**

| | 5'-RACE | GAPDH (positive control) | UPM alone (negative control) | GSP alone (negative control) |
|---|---|---|---|---|
| 5'-RACE c DNA | 2.5 | 2.5 | 2.5 | 2.5 |
| UPM (10x) | 5.0 | 5.0 | 5.0 | |
| GSP (10 µM) | 1.0 | | | 1.0 |
| Control 5'-RACE GAPDH primer (10 µM) | | 1.0 | | |
| H₂O | | | 1.0 | 5.0 |
| Master mix | 41.5 | 41.5 | 41.5 | 41.5 |
| Final volume | 50.0 | 50.0 | 50.0 | 50.0 |

RT-PCR was carried out by a standard method. That is, a master mix was added to 2 µL of cDNA, 1 µL of 10 µM primer, and 4.5 µL of H₂O to give 50 µL of a reaction mixture, which was reacted under conditions of 94°C 3 minutes; 30 cycles of 94°C 3 seconds and 65°C 3 minutes; 65°C 3 minutes. The cDNA used was that prepared for the above-mentioned 5'RACE. As RT-PCR primer, 1941, 1942, 1943, 1944 (43C5, 72-5 heavy chain constant region), 2001, 2003, 2004, 2006 (44B1 heavy chain constant region), 1937, 1938 (43C5, 72-5, 44B1 light chain constant region), and 1980, 1982, 1999, 2000, 2007, 2009 (72-5 light chain variable region) were used.

Amplification products (cDNA) of the variable region and the constant region of each antibody gene obtained as above were confirmed by electrophoresis with 2% agarose gel, a band with a predicted molecular weight was excised, purified by a glass bead method, and subcloned to TOPO-TA cloning vector plasmid. With regard to the 44B1 heavy chain variable region, in order to eliminate a pseudogene containing a transposon, the 5'-RACE product was digested with an XbaI specific to the transposon region, two bands of about 700bp (44B1VH-700) and 800bp (44B1VH-800), whose molecular weight did not change, were excised from the agarose gel, and subcloning was carried out.

### (2) Sequencing

The plasmid obtained above was purified by a miniprep method in accordance with the manual of a BigDye (registered trademark) Terminator Cycle Sequencing Kit. That is, 400 ng of plasmid DNA was mixed with 4 µL of BigDye (registered trademark), 2 µL of 5x sequencing buffer, 3.2 µL of sequencing primer (1 pM/ µL), and an appropriate amount of deionized water to give 20 µL, and a PCR reaction was carried out in a thermal cycler (Perkin-Elmer GeneAmp (registered trademark) System 2400) (35 cycles: 96°C 10 seconds, 55°C 5 seconds, 60°C 4 minutes). Subsequently, desalination was carried out by means of a Performa DTR gel filtration cartridge, solvent was removed by means of a SpeedVac, 20 µL of Hi-Di formamide was added, and the mixture was heated at 95°C for 5 minutes then rapidly cooled in iced water for 2 minutes to give a sequence sample. Sequencing was carried out using an ABI PRISM 3100.

From the results, 6 types of sequences, that is, 43C5 heavy chain (43C5H: SEQ ID NO: 1), 43C5 light chain (43C5L: SEQ ID NO: 2), 72-5 heavy chain (72-5H: SEQ ID NO: 3), 72-5 light chain (72-5L: SEQ ID NO: 4), 44B1 heavy chain (44B1H: SEQ ID NO: 5), and 44B1 light chain (44B1L: SEQ ID NO: 6) were obtained. Table 6 shows the signal peptide region, the variable region, and the constant region of each sequence by base number.

### [Table 6]

**Table 6 Constitution of anti-prion antibody gene**

| | Signal peptide region | Variable region | Constant region |
|---|---|---|---|
| 43C5H | 116-172 | 173-527 | 528-1501 |
| 43C5L | 111-167 | 268-505 | 506-827 |
| 72-5H | 74-130 | 131-476 | 477-1450 |
| 72-5L | 38-94 | 95-426 | 427-748 |
| 44B1H | 149-205 | 206-559 | 560-1552 |
| 44B1L | 90-149 | 150-397 | 398-806 |

### Example 4: Anti-prion antibody gene expression

### (1) Cloning to expression vector

With regard to IgG1 heavy chains of 43C5 and 72-5, the constant region was excised at 2 parts, that is, the EcoRI/XmaI site and the XmaI/BglII site. Subsequently, the expression vector plasmid pCAcc was digested with EcoRI/BglII, and into this was inserted the constant region (CH1-2) EcoRI/XmaI and the constant region (CH2-3) XmaI/BglII by 3 part ligation, thus preparing pCAcc/CH. Subsequently, this was digested with EcoRI/BstEII, and the cDNA of the variable region excised at the EcoRI/BstEII site was ligated at the EcoRI/BstEII site, thus giving expression plasmids pCAcc/43C5H and pCAcc/72-5H.

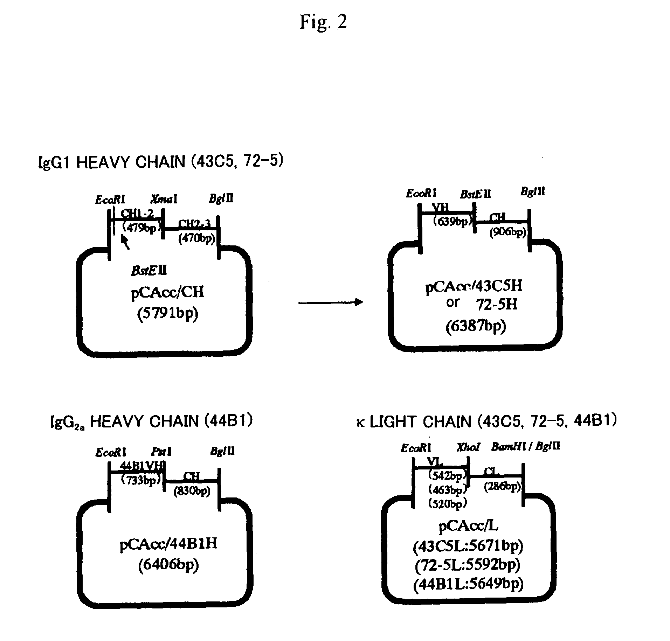

With regard to κ light chains of 43C5 and 44B1, the variable region was excised at the EcoRI/XhoI site, and the constant region was excised at the XhoI/BamHI site. These insert cDNAs were inserted into pCAcc/EcoRI/BglII by 3 part ligation, thus giving expression plasmids pCAcc/43C5L and pCAcc/44B1L.
With regard to 72-5κ light chain, the pCAcc/43C5L prepared above was digested with EcoRI/XhoI, and into this was inserted the variable region excised at the EcoRI/XhoI site, thus giving expression plasmid pCAcc/72-5L.
With regard to 44B1IgG₂ₐ heavy chain, the variable region was excised at the EcoRI/PstI site, the constant region was excised at the PstI/BglII site, and they were inserted into pCAcc/EcoRI/BglII by 3 part ligation, thus giving expression plasmid pCAcc/44B1H (see FIG. 2).

### (2) Production of antibody

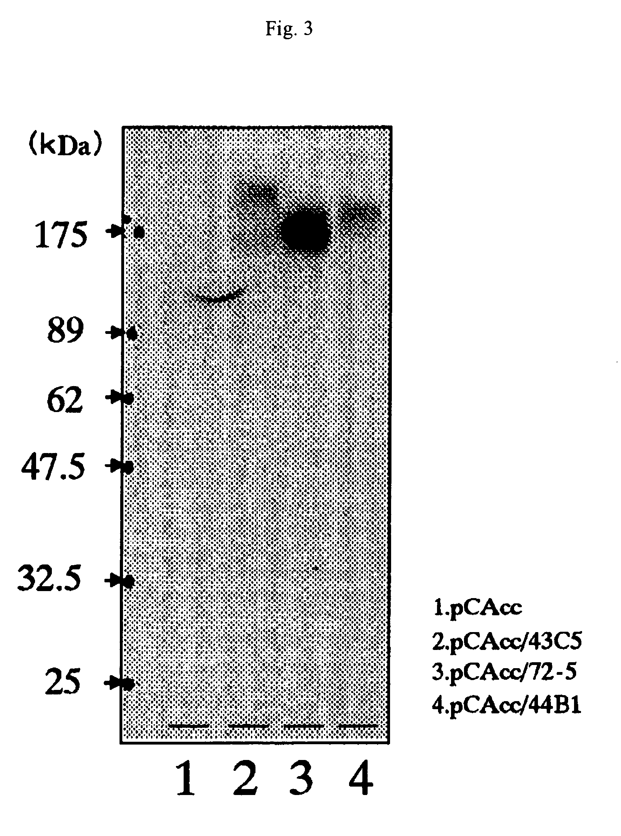

293T cells were cultured on 6-well plate at 5 × 10⁵ cells/well for 12 hours. The expression plasmids obtained above were purified by a miniprep method to give 2.5 µg each of expression plasmids for the heavy chain and the light chain of each antibody, which were cotransfected to 293T cells by a lipofection method in accordance with the lipofectAMINE (registered trademark) 2000 protocol. After culturing for 48 hours, 293T cells were collected, solubilized, and then subjected to SDS-PAGE under reducing conditions. Migrated gels were transferred on a nitro cellulose membrane, and expression of an antibody was confirmed by a horseradish-labeled anti-mouse IgG antibody. As shown in FIG. 3, antibody protein with a molecular weight of 175 to 180 kDa was detected under reducing conditions.

In another experiment, an antibody secreted by transfected 293T cells was quantitatively measured by a sandwich ELISA method. 293T cells were transfected with the expression plasmid of each antibody in the same manner as above and cultured for 48 hours, and the supernatant was subsequently collected. As an immobilizing antibody that was to be bound to a plate, anti-mouse IgG antibody was used. 50 µL of the immobilizing antibody, whose concentration was adjusted with 0.1 M carbonate buffer (pH 8.5) so as to give a predetermined concentration, was added to each well and incubated at 37°C for 1 hour, thus making it bind to the plate surface. Subsequently, in order to mask non-antigen-binding sites of the plate surface, 150 µL/well of goat serum (blocking agent) was added, and a blocking operation was carried out at 37°C for 1 hour. After the blocking operation, each well was washed with 0.02% Tween (registered trademark)-PBS for 5 minutes 3 times to remove excess blocking agent.

The culture supernatant and mouse IgG for a calibration curve were diluted to give predetermined concentrations, and 50 µL thereof was added to each well of the plate after blocking. A reaction was carried out at 37°C for 1 hour, and each well was washed with 0.02% Tween (registered trademark)-PBS for 5 minutes 3 times. Subsequently, as a secondary antibody, the horseradish-labeled anti-mouse IgG antibody was diluted with goat serum to give a predetermined concentration and added. After a reaction was carried out at 37°C for 1 hour, the wells were washed, 100 µL of a colorimetric substrate (TMB) was added to each well and allowed to stand at 37°C for 15 minutes, following this 50 µL/well of 1 N H₂SO₄, which is a reaction terminating solution, was added, and measurement was carried out at 450 nm. From the results, it was shown that an expression antibody was secreted in the culture supernatant of cells transfected with 44B1, 72-5, and 43C-5 antibody genes (see Table 7).

### [Table 7]

**Table 7**

| Quantitative analysis of anti-PrP antibody secreted by transfected cells | | | |
|---|---|---|---|
| Clone name | 44B1 | 72-5 | 43C-5 |
| Antibody concentration (µg/mL) | 230 | 260 | 10 |

### (3) Reactivity of recombinant antibody

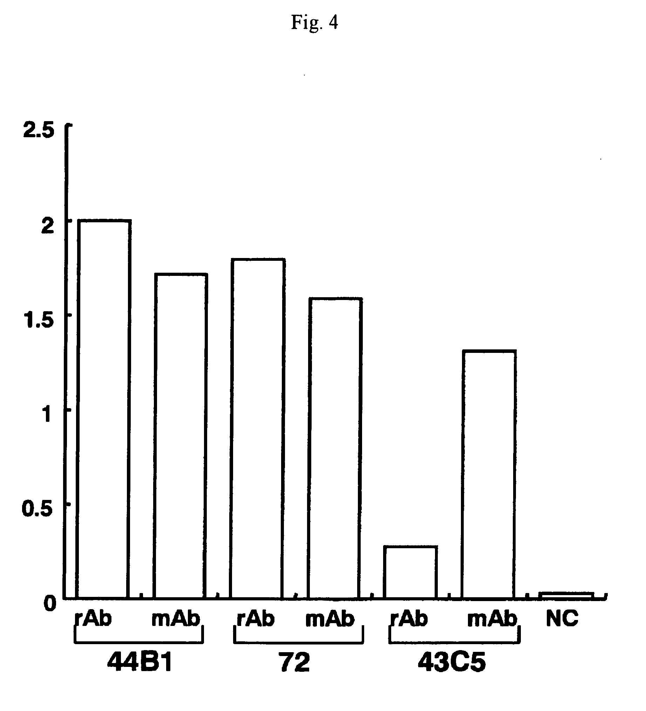

The reactivity of antibody secreted by a transfected cell toward prion protein was examined by a sandwich ELISA method. As shown in FIG. 4, it was confirmed that the recombinant antibody reacted with prion protein.

### Example 5: Preparation of anti-prion antibody gene-containing adenovirus

### (1) Architecture of anti-prion antibody gene-containing cosmid

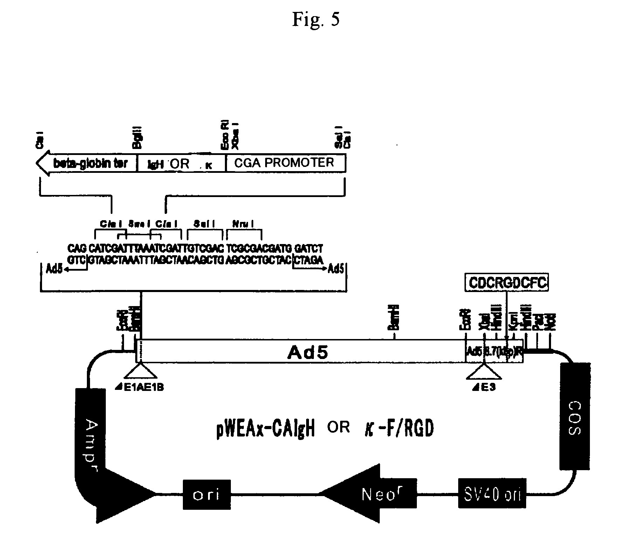

With regard to insert cDNA, antibody gene cDNA was excised from the pCAcc/IgH (pCAcc/43C5H, 72-5H, and 44B1H) and pCAcc/L (pCAcc/43C5L, 72-5L, and 44B1L) obtained in Example 4 at the ClaI site, and purified by a glass milk method. As a cosmid vector, pWEAx-F/RGD/ClaI/CIAP expressing an RGD fiber-modified adenovirus was used (Nakamura T et al. 2002. Hum Gene Ther 13(5): 613-26). A ligation reaction was carried out by adding the insert cDNA to 1 µg of the vector at a molar ratio in the range of 1:1 to 10:1. Subsequently, 6 types of cosmids (pWEAx-43C5HL-F/RGD, pWEAx-43C5LL-F/RGD, pWEAx-72-5HL-F/RGD, pWEAx-72-5LL-F/RGD, pWEAx-44B1HL-F/RGD, pWEAx-44B1LL-F/RGD) containing each of the above-mentioned cDNAs were prepared using a Gigapack III Gold Packaging kit (manufactured by Invitrogen) (see FIG. 5). The cosmids were subjected to a DNA check by a restriction enzyme, then cultured on a 500 mL scale, and purified with a NucleoBond BAC100.

### (2) Preparation of anti-prion antibody gene-containing adenovirus

Adenovirus containing the heavy chain gene or the light chain gene of each anti-prion antibody was prepared by a single cosmid method or a COS-TPC method.

### 1) Single cosmid method

20 µg of the recombinant cosmid DNA obtained in Example 5 (1) was digested with PacI. The digest was extracted with phenol/chloroform, precipitated from ethanol, and dissolved in 20 µL of sterile distilled water. 5 µg of the PacI-digested cosmid thus obtained was transfected into 293T cells cultured in a 10 cm petri dish by a lipofection method. Culturing was carried out at 37°C with 5% CO₂ overnight, and cells were collected using EDTA-PBS(-). The original solution of the collected cell suspension, a 3 times dilution, and a 5 times dilution were sown on a collagen coated 96-well plate.

### 2) COS-TPC method

5 µg of the recombinant cosmid DNA and 1 µg of the adenovirus genome DNA-TPC were transfected into 293T cells by a calcium phosphate method. Culturing was carried out at 37°C with 5% CO₂ overnight, and cells were collected using EDTA-PBS(-). The original solution of the collected cell suspension, a 3 times dilution, and a 5 times dilution were sown on a collagen coated 96-well plate.

### 3) Purification of adenovirus

After five days and ten days, 50 µL of 10% FBS-DMEM was added to each of the wells prepared above. On the 7th day and thereafter, wells in which the virus had grown and the cells showed cytopathic changes were checked. A few days after this, the cells were collected together with the culture solution from a well in which all the cells showed cytopathic changes. The cells thus collected were subjected to centrifugation after ultrasonic disintegration, and the supernatant was used as a primary virus solution.
293T cells that had been cultured up to 70 to 100% confluent were prepared in a collagen-coated 24-well plate. 2 wells of 293T cells were infected with each sample of the primary virus solution at 10 µL/well. A few days after this, the cells were collected together with the culture solution from a well in which all the cells showed cytopathic changes. Part of the cell solution thus collected was subjected to centrifugation after ultrasonic disintegration, and the supernatant was used as a secondary virus solution. The rest of the cell solution was subjected to centrifugation and the medium was discarded to give a cell pack; after the total DNA was extracted, it was digested with a restriction enzyme, and the structure of the recombinant adenovirus DNA was confirmed.

With regard to the secondary virus solution, 293T cells were prepared in a 10 cm petri dish (90% confluent) and infected with 30 µL of the secondary virus solution. After 3 to 4 days, it was confirmed that all the cells showed cytopathic changes, and the cells were collected together with the culture solution. Part of the cell solution thus collected was used as a cell pack; after the total DNA was extracted, it was digested with a restriction enzyme, and the structure of the recombinant adenovirus DNA was confirmed. The rest of the cell solution was subjected to centrifugation after ultrasonic disintegration, and the supernatant was used as a tertiary virus solution and stored at -80°C. With regard to the tertiary virus solution, 30 plates of 293T cells in a 10 cm petri dish (90% confluent) were subsequently prepared and infected with 30 µL of the tertiary virus solution. After 3 to 4 days, it was confirmed that all the cells showed cytopathic changes, and the cells were collected together with the culture solution. After ultrasonic disintegration, virus purification was carried out by a CsCl step gradient method. It was confirmed that the purified virus was not contaminated with wild-type adenovirus by a DNA check using a restriction enzyme and PCR, and it was stored as a purified virus at -80°C.
In this way, 6 types of adenovirus vectors (Ax43C5H, Ax43C5L, Ax72-5H, Ax72-5L, Ax44B1H, and Ax44B1L) containing each of the above-mentioned cDNAs were obtained.

### Example 6: Preparation of mesenchymal stem cells (MSC)

### (1) Preparation of mouse MSC

A thighbone was excised from a mouse (ICR or C57BL/6 strain, 6 weeks old, female) under deep anesthesia; after surplus muscle tissue was removed in PBS-, the femoral head and a part immediately above the knee joint were cut off, and bone-marrow cells were washed out with 2 mL of a medium (2% FBS, penicillin/streptomycin, L-glutamine-containing IMDM) in a 10 cm dish. The bone-marrow cells thus obtained were collected in a 50 mL tube (manufactured by Falcon) via a 70 µm mesh, washed with PBS (1000 rpm, 10 minutes), then suspended again in a culture medium (20% heat-inactivated CS or FBS, 50 µM 2-ME, penicillin/streptomycin, L-glutamine-containing IMDM), and sown on a 6-well plate (1 thighbone/well). After the cells were cultured at 33°C with 5% CO₂ for 24 hours, floating cells were removed, and adherent cells were further cultured. Thereafter, the medium was replaced every 3 to 4 days. Subculturing was carried out by adding 1 mL of trypsin/EDTA, allowing it to stand at room temperature for 5 minutes, and then collecting easily detached cells.

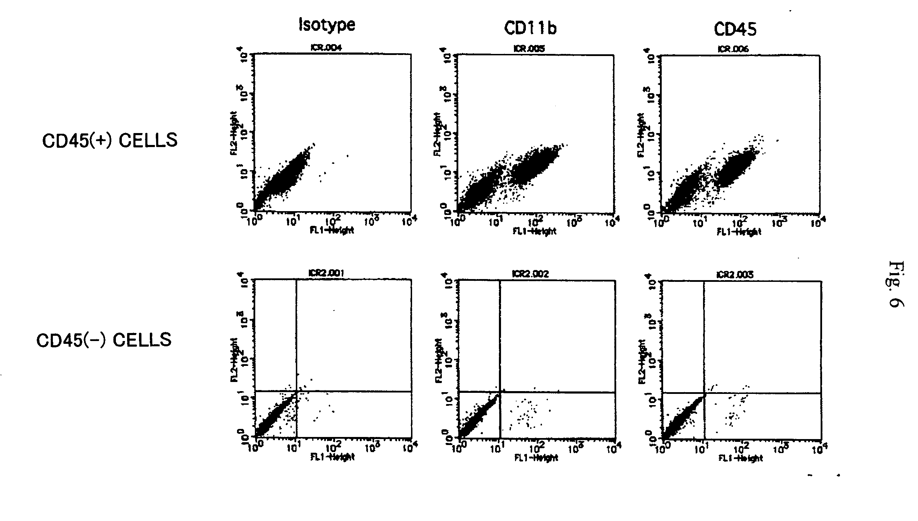

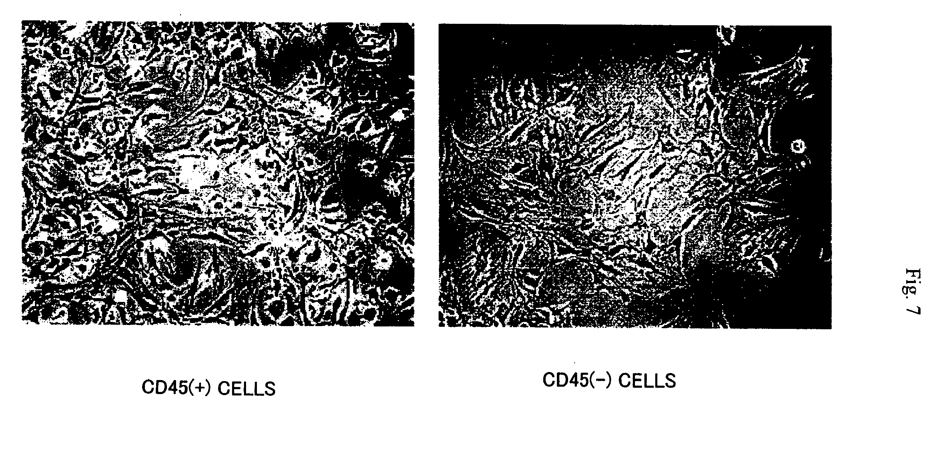

Subsequently, among the above-mentioned adherent cells, CD45- cells were selected as follows. Cells on the 14th (to 21st) day of culturing were first collected, washed with 2% FCS-containing IMDM 3 times, and resuspended in 2% FCS-containing IMDM at a concentration of 1 × 10 ⁷ cells/mL. The cell suspension thus obtained (1 mL) was mixed with 10 µL of CD45 MicroBead liquid, and incubation was carried out at room temperature for 20 minutes. Subsequently, CD45- cells were separated by a magnetic cell separation method employing an AutoMACS (registered trademark) (manufactured by Miltenyi Biotec). It was confirmed by FACS analysis (FIG. 6) and morphological observation (FIG. 7) that the cells thus obtained were CD11b- and CD45- mesenchymal stem cells (MSC). From FIG. 7, it was found that a colony of CD45+ cells was a mixture of small cells and large, relatively broad cells, whereas a colony of CD45- cells was a single cell population of large broad cells alone, which is characteristic of mesenchymal stem cells.

### (2) Preparation of human MSC

Human MSC was prepared in accordance with the description of Kobune M et al. 2003. Experimental Haematology 31: 1-8. That is, bone marrow mononuclear cells were collected from the posterior iliac crest of a healthy human volunteer, and incubated in a 150 cm² tissue culturing plastic flask overnight. Floating cells were subsequently removed by washing, and adherent cells were cultured in a culture medium (10% heat inactivated fetal bovine serum-containing DMEM) at 37°C with 5% CO₂ under a humid environment.

### Example 7: Introduction of anti-prion antibody gene into MSC

### (1) Introduction by adenovirus vector

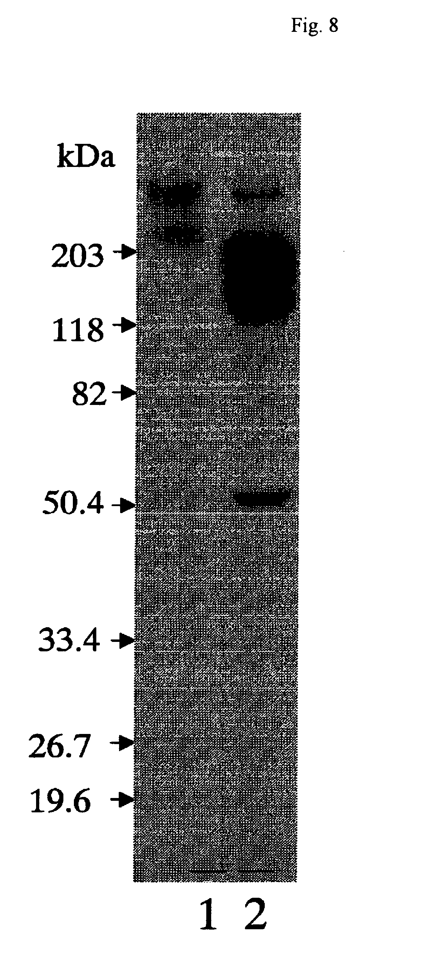

ICR mouse-derived MSC (ICR/MSC) was sown on a 24-well plate at a concentration of 5 × 10⁴ cells/well, and Ax43C5H and Ax43C5L or Ax44B1H and Ax44BlL obtained in Example 5 were each added thereto at a concentration of 1500 pu/cell, thus carrying out coinfection. After culturing for 48 hours, the presence of antibody in the culture supernatant was evaluated by the Western blot method. The results obtained for 43C5 are shown in FIG. 8. Furthermore, the amount of antibody in the culture supernatant was quantitatively analyzed by a sandwich ELISA method in the same manner as in Example 4. The results are given in Table 8 below.

### (2) Introduction by electroporation

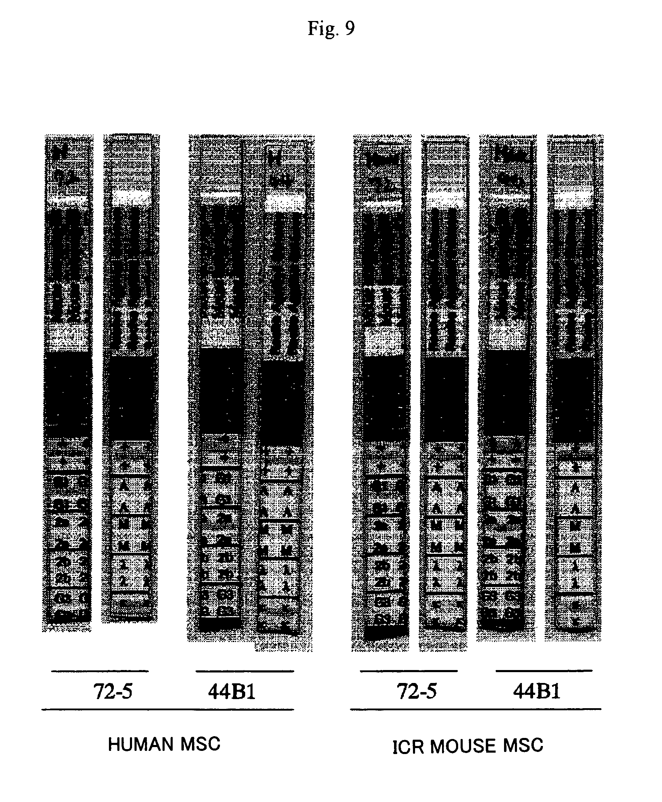

2.5 µg each of the expression plasmids pCAcc/72-5H and pCAcc/72-5L, or pCAcc/44B1H and pCAcc/44B1L were added to a cell suspension containing ICR mouse or human MSC at a concentration of 2 × 10⁵ cells/100 µL, and electroporation was carried out using a human MSC nucleofector kit (manufactured by Amaxa Biosystems) in accordance with the manufacturer's protocol under U-23 high transfection efficiency conditions. Cells thus obtained were sown on a 6-well plate, cultured for 48 hours, part of the culture supernatant was collected, and expression of the antibody was confirmed by an IsoStrip (manufactured by Roche Diagnostics) (FIG. 9). Following this, culturing was carried out for a further 48 hours, and the amount of antibody in the culture supernatant was quantitatively analyzed by a sandwich ELISA method in the same manner as in Example 4. The results are given in Table 8 below.

### [Table 8]

**Table 8 Secretion of anti-prion antibody by MSC into which an anti-prion antibody gene has been introduced (unit: µg/10⁵ cells/48 hours)**

| | Adenovirus | Electroporation | |
|---|---|---|---|
| | ICR/MSC | ICR/MSC | Human MSC |
| 43C5 | 4 | | |
| 72-5 | | 0.63 | 2.8 |
| 44B1 | 0.15 | 0.28 | 2.8 |

### Example 8: Preparation of agent for delivering substance to lesion site of prion disease

### (1) Introduction of LacZ gene to mesenchymal cells

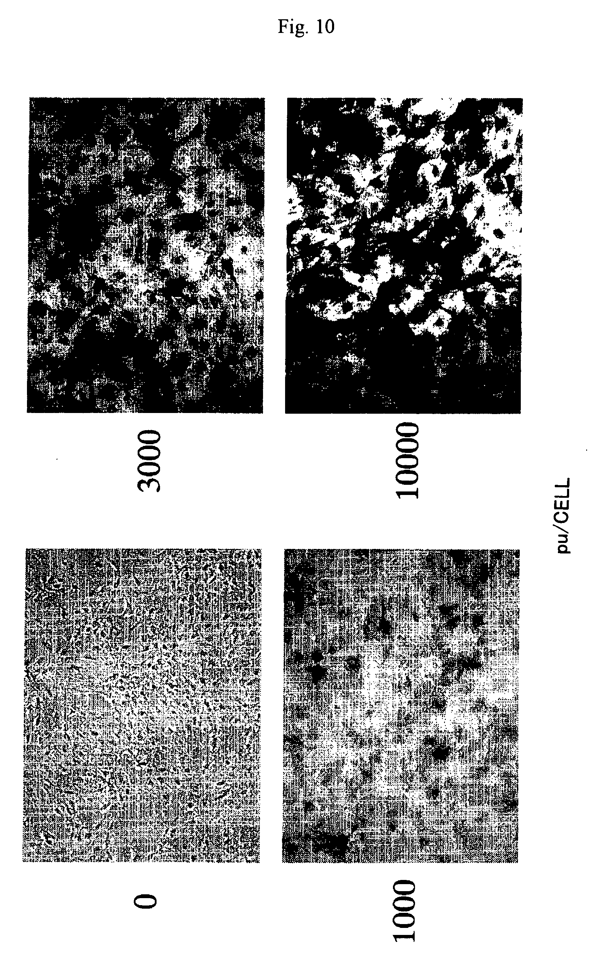

ICR/MSC prepared in Example 6 (1) was sown on a 24-well plate at a concentration of 5 × 10³ cells/well, and cultured at 33°C for 12 hours with 5% CO₂. Infection was carried out with adenovirus vector AxCAZ3-F/RGD expressing LacZ gene at concentrations of 0, 1000, 3000, and 10000 pu/cell at 37°C for 1 hour, and after 48 hours X-gal staining was carried out. From the results, it was confirmed that at a concentration of 3000 pu/cell or greater, the LacZ gene was introduced into all of the cells (FIG. 10).

### (2) Introduction of DsRed gene into mesenchymal cell

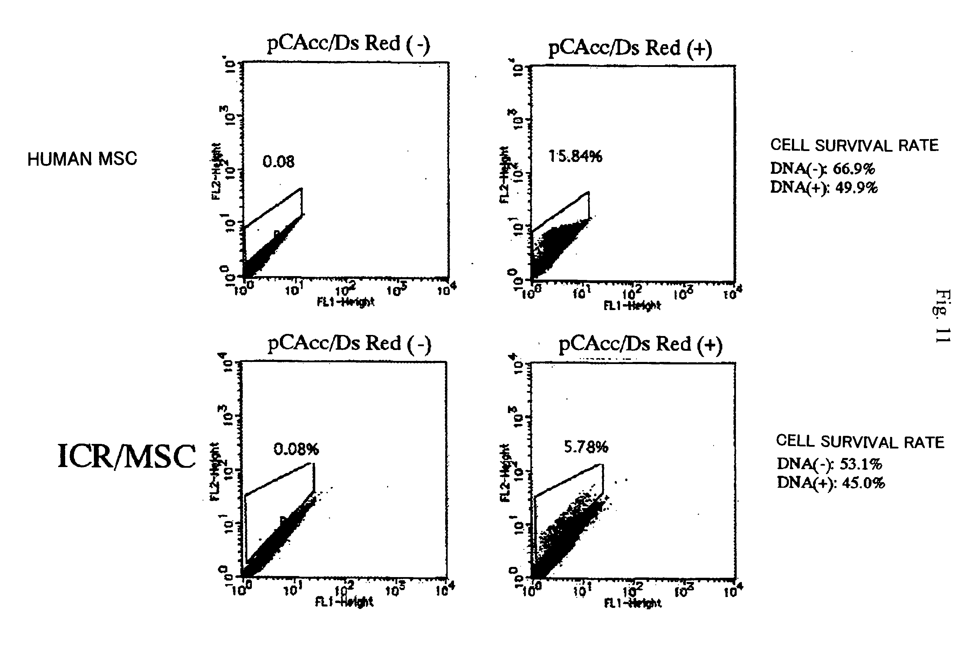

Introduction of DsRed gene was carried out using a Nucleofector (manufactured by Amaxa Biosystems) Human MSC nucleofector kit. That is, 2 × 10⁵ counts of ICR/MSC prepared in Example 6 (1) or human MSC prepared in Example 6 (2) were suspended in 100 µL of a transfection buffer, 2 µg of pCAcc/DsRed expression vector containing DsRed gene was added, and electroporation (program: U-23 high transfection efficiency) was carried out. Cells thus obtained were sown on a 6-well plate and cultured for 48 hours, and the gene introduction efficiency was then examined by FACS. From the results, it was confirmed that a good gene introduction efficiency of 15.84% for human MSC and 5.78% for ICR/MSC was obtained (FIG. 11).
Since the MSC obtained in (1) and (2) above both can migrate to a lesion site of a prion disease, a desired gene such as LacZ gene or DsRed gene or a gene product thereof can be delivered to the site.

### Example 9: Spongiform encephalopathy inhibiting effect by anti-prion antibody

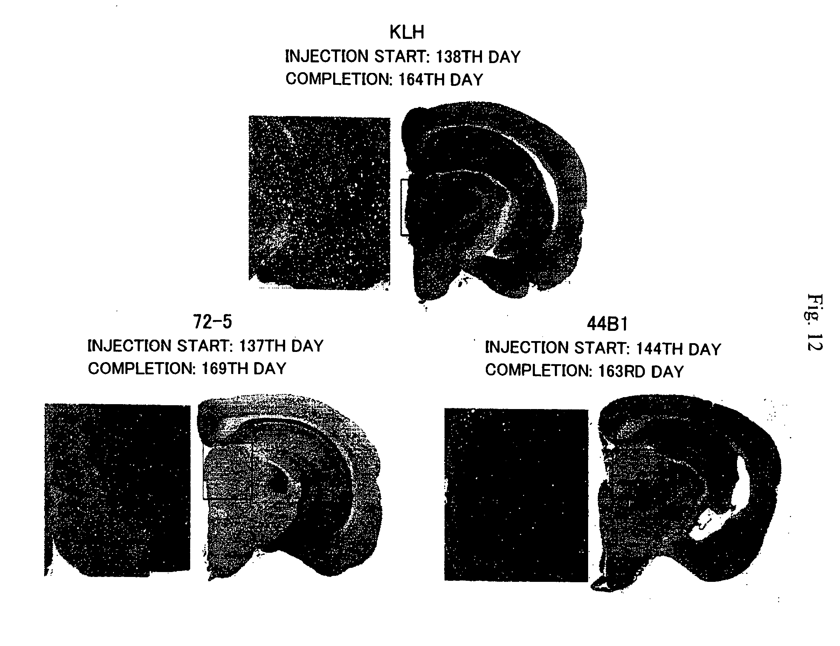

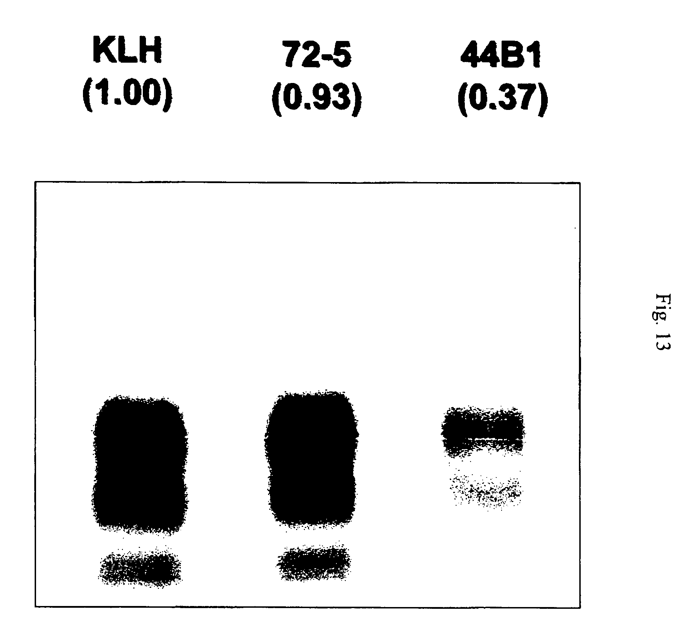

The spongiform encephalopathy inhibiting effect of anti-prion antibodies 72-5 and 44B1 was evaluated using a prion disease model mouse. The subjects were 4 week old female ICR mice (CLEA Japan, Inc.). 2 µL of 10% brain homogenate of the scrapie Obihiro strain (obtained from Hokkaido University) was intracerebrally administered to the subjects. According to this procedure, PrP^{sc} is normally detected in the brain after about 60 to 90 days (60 days and thereafter in the Western blot method, 90 days and thereafter in the immunohistochemical method). All of the subjects exhibited typical symptoms of spongiform encephalopathy such as ataxia before 125 days after administration. On the 137th to 144th day after administration, which is clinically classified as midterm, 72-5, 44B1, or KLH antibodies were injected into the dorsal third cerebroventricle of separate subjects using an Alzet (registered trademark) osmotic pump (200 µL/week, 28 µg/mouse/day). KLH means anti-KLH (Keyhole Limpet Haemocyanin) antibody (Fujirebio Inc.) and was used as a negative control. When the negative control exhibited late stage clinical symptoms such as emaciation or astasia, the subject was euthanized, and a brain tissue sample was collected. The tissue thus sampled was subjected to histological analysis and the Western blot method, and the state of the brain tissue and the amount of PrP^{Sc} accumulated were evaluated (the results are shown in FIG. 12 and FIG. 13).

As is clear from FIG. 12, a large number of cavities characteristic of spongiform encephalopathy were observed in the brain tissue (thalamus) of the subject to which KLH as a negative control was administered, whereas such cavities were hardly observed in similar brain tissue of subjects to which 72-5 or 44B1 was administered. Furthermore, the amount of PrP^{Sc} accumulated in the brain tissue of the subjects to which 72-5 or 44B1 was administered was clearly reduced compared with the subject to which KLH was administered (see FIG. 13). It has therefore become clear that an anti-prion antibody such as 72-5 and 44B1 prevents the accumulation of PrP^{Sc} and can suppress the occurrence of spongiform encephalopathy disease.

### Example 10: Survival of MSC

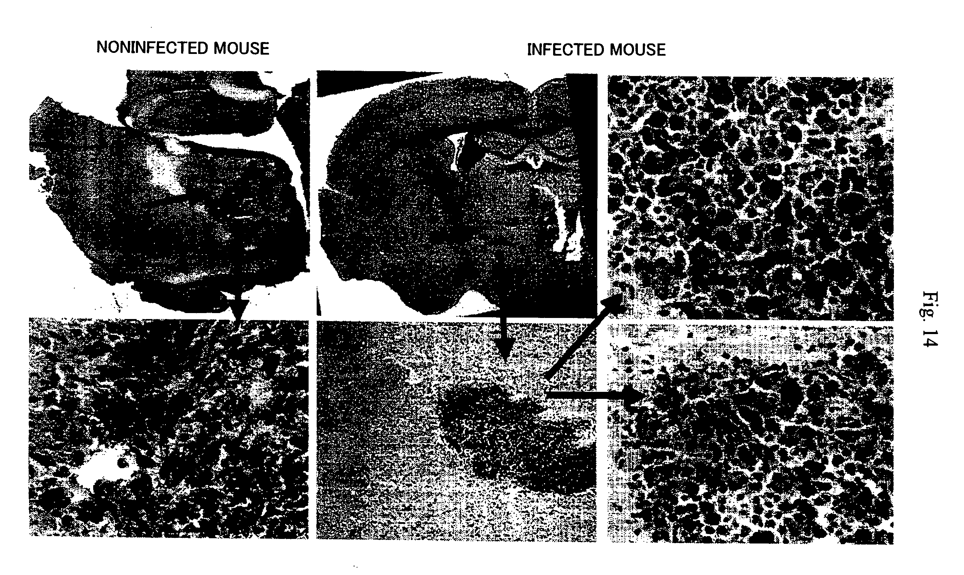

The ability of MSC to survive in the brain was evaluated using a prion disease model mouse. As subjects, an ICR mouse (infected mouse) that had been subjected to the procedure described in Example 9 and an ICR mouse (noninfected mouse) as a control that had not been subjected to such a procedure were used. On the 140th day after administration of PrP^{Sc}, 1 × 10⁴ counts of LacZ-expressing MSC prepared in Example 8 were implanted in the thalamus of the subject. On the 142nd day after administration of PrP^{sc} the mouse was euthanized, the brain tissue was stained with X-gal, and the distribution of MSC was evaluated. As shown in FIG. 14, it was found that in both the subjects, the implanted MSC survived in the implanted area. From the results of Examples 9 and 10, it can therefore be expected that the prion disease will be improved by implanting the anti-prion antibody-secreting MSC obtained in Example 7 in a prion disease model mouse.

### Example 11: Preparation of mouse-human chimera antibody

Based on the nucleic acid sequence that codes for each of the mouse anti-prion antibodies (43C5, 72-5, and 44B1) obtained in Example 3, the antibody constant region thereof was replaced with a human-derived one to give mouse-human chimera antibodies (m/h43C5, m/h72-5, and m/h44B1). A nucleic acid that codes for the human antibody constant region was obtained by a method described in Shibagaki et al. 1998 Eur J Immunol 28: 1125-1133, and Shibagaki et al. 1998 Eur J Immunol 29: 4081-4091. That is, a desired nucleic acid was obtained by an RT-PCR method using RNA from a myeloma cell line producing human-mouse chimera monoclonal antibody L6 as a template, SEQ ID NO: 28 oligonucleotide as a forward primer, and SEQ ID NO: 29 oligonucleotide as a reverse primer (ref. Linsley et al. 1991 J Exp Med 173: 721-30). By linking a nucleic acid that codes for the signal peptide region and the variable region of each mouse antibody shown in Table 6 to the upstream of the nucleic acid obtained above, a nucleic acid that codes for the mouse-human chimera antibody was obtained, and its sequence was determined. The nucleic acid sequence and the amino acid sequence that code for each antibody were as shown in Table 9 below.

**[Table 9]**

| | Nucleic acid sequence | Amino acid sequence |
|---|---|---|
| m/h43C5 heavy chain | SEQ. ID. NO: 30 | SEQ. ID. NO: 36 |
| m/h43C5 light chain | SEQ. ID. NO: 31 | SEQ. ID. NO: 37 |
| m/h72-5 heavy chain | SEQ. ID. NO: 32 | SEQ. ID. NO: 38 |
| m/h72-5 light chain | SEQ. ID. NO: 33 | SEQ. ID. NO: 39 |
| m/h44B1 heavy chain | SEQ. ID. NO: 34 | SEQ. ID. NO: 40 |
| m/h44B1 light chain | SEQ. ID. NO: 35 | SEQ. ID. NO: 41 |

The above-mentioned nucleic acid can be expressed by, for example, the same procedure as in Example 4, or expressed by introduction into MSC by the same method as in Example 7.

### Example 12: Effect of MSC cells in improving prion disease and migratory capacity to affected area

For example, 1 × 10⁴ counts of anti-prion antibody gene-expressing MSC obtained in Example 7 or LacZ-expressing MSC obtained in Example 8 are intravenously administered to prion disease model mice described in Example 9 after the spongiform encephalopathy disease occurred (at least 125 days after inoculation with scrapie Obihiro strain). After 2 to 20 days, some of the mice areeuthanized, the brain tissue is sampled in the same manner as in Example 9 and subjected to histological analysis and the Western blot method, and the state of the mouse brain tissue and the amount of PrP^{sc} accumulated are evaluated. The rest of the mice are observed with respect to changes in clinical state. This enabled the effect of MSC cells in improving the prion disease to be easily confirmed. Furthermore, the brain tissue sampled above is subjected to X-gal staining in the same manner as in Example 10, thus examining the distribution of MSC, and this enabled the capacity of the MSC cells to migrate to the area affected by the prion disease to be confirmed.

### Example 13: Effect of anti-prion antibody gene-containing vector and anti-prion antibody-secreting MSC cells in improving prion disease

Anti-prion antibody gene-containing adenovirus (e.g. 5 × 10⁸ particles) obtained in Example 5 or anti-prion antibody gene-expressing MSC (e.g. 1 × 10⁴ counts) obtained in Example 7 is intracerebrally administered to a prion disease model mouse described in Example 9 after spongiform encephalopathy disease occurred (at least 125 days after inoculation with scrapie Obihiro strain). After 2 to 20 days, some of the mice are euthanized, the brain tissue is sampled in the same manner as in Example 9 and subjected to histological analysis and the Western blot method, and the state of mouse brain tissue and the amount of PrP^{Sc} accumulated are evaluated. The rest of the mice are observed with respect to changes in clinical state. This enabled the effect of the adenovirus or MSC cells in improving the prion disease to be easily confirmed.

## Claims

1. An agent for the treatment of a prion disease, the agent comprising mesenchymal cells as an effective component.

2. The agent according to Claim 1, wherein the mesenchymal cells have abnormal prion growth inhibitory activity.

3. The agent according to either Claim 1 or 2, wherein the mesenchymal cells have introduced thereinto an anti-prion antibody gene having abnormal prion growth inhibitory activity.

4. The agent according to Claim 3, wherein the antibody gene comprises an antibody heavy chain gene and an antibody light chain gene,
the antibody heavy chain gene being selected from the group consisting of
(1a) a nucleic acid that contains a sequence selected from the group consisting of SEQ ID NOS: 1, 3, 5, 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for,
the antibody light chain gene being selected from the group consisting of
(2a) a nucleic acid containing a sequence selected from the group consisting of SEQ ID NOS: 2, 4, 6, 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for, and
an antibody obtained by expression of the antibody heavy chain gene and the antibody light chain gene having abnormal prion growth inhibitory activity.

5. The agent according to any one of Claims 1 to 4, wherein it is for intravenous administration.

6. The agent according to any one of Claims 1 to 5, wherein the mesenchymal cells are selected from the group consisting of bone-marrow cells, umbilical cord blood cells, and peripheral blood cells.

7. An anti-prion antibody gene comprising an antibody heavy chain gene and an antibody light chain gene,
the antibody heavy chain gene being selected from the group consisting of
(1a) a nucleic acid that contains a sequence selected from the group consisting of SEQ ID NOS: 1, 3, 5, 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for,
the antibody light chain gene being selected from the group consisting of
(2a) a nucleic acid containing a sequence selected from the group consisting of SEQ ID NOS: 2, 4, 6, 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for, and
an antibody obtained by expression of the antibody heavy chain gene and the antibody light chain gene having abnormal prion growth inhibitory activity.

8. A vector comprising the anti-prion antibody gene according to Claim 7.

9. The vector according to Claim 8, wherein it is an adenovirus vector containing an RGD sequence.

10. An anti-prion chimera antibody comprising an antibody variable region that is coded for by the anti-prion antibody gene according to Claim 7, and an antibody constant region of an animal other than a mouse.

11. The anti-prion chimera antibody according to Claim 10, wherein it is coded for by
an antibody heavy chain gene selected from the group consisting of
(1a) a nucleic acid that contains a sequence selected from the group consisting of SEQ ID NOS: 30, 32, and 34,
(1b) a nucleic acid that, by genetic code degeneration, codes for the same polypeptide as does the nucleic acid of (1a) above,
(1c) a nucleic acid that has a variation in the sequence of the nucleic acid of (1a) or (1b) above but that still codes for a polypeptide that has the same function as the polypeptide that said nucleic acid codes for, and
(1d) a nucleic acid that hybridizes under stringent conditions with a complementary strand of the nucleic acid of any one of (1a) to (1c) above or a fragment thereof, and that codes for a polypeptide that has the same function as the polypeptide that said nucleic acid codes for, and
an antibody light chain gene selected from the group consisting of
(2a) a nucleic acid containing a sequence selected from the group consisting of SEQ ID NOS: 31, 33, and 35,
(2b) a nucleic acid that, by genetic code degeneration, codes for the same polypeptide as does the nucleic acid of (2a) above,
(2c) a nucleic acid that has a variation in the sequence of the nucleic acid of (2a) or (2b) above but that still codes for a polypeptide that has the same function as the polypeptide that said nucleic acid codes for, and
(2d) a nucleic acid that hybridizes under stringent conditions with a complementary strand of the nucleic acid of any one of (2a) to (2c) above or a fragment thereof, and that codes for a polypeptide that has the same function as the polypeptide that said nucleic acid codes for.

12. A nucleic acid that codes for the anti-prion chimera antibody according to either Claim 10 or 11.

13. A method of producing cells having abnormal prion growth inhibitory activity, the method comprising introducing into cells a gene that imparts abnormal prion growth inhibitory activity.

14. The method according to Claim 13, wherein the gene that imparts abnormal prion growth inhibitory activity is an anti-prion antibody gene.

15. A method of producing cells having abnormal prion growth inhibitory activity, the method comprising introducing the gene according to Claim 7 into cells via the vector according to either Claim 8 or 9.

16. The method according to any one of Claims 13 to 15, wherein the cells are mesenchymal cells.

17. Cells produced by the method according to any one of Claims 13 to 16, the cells having abnormal prion growth inhibitory activity.

18. A method of producing an agent for the treatment of a prion disease, the method comprising the method according to Claim 16.

19. A sustained-release preparation for the treatment of a prion disease, the preparation releasing an anti-prion antibody.

20. The preparation according to Claim 19, wherein the anti-prion antibody is an antibody that is coded for by the nucleic acid according to Claim 7 and/or the anti-prion chimera antibody according to either Claim 10 or 11.

21. The preparation according to Claim 19 or 20, wherein it is in osmotic pump form.

22. The preparation according to either Claim 19 or 20, wherein it comprises anti-prion antibody-secreting cells.

23. The preparation according to Claim 22, wherein the anti-prion antibody-secreting cells are the cells according to Claim 17.

24. A method of treating a prion disease, the method comprising administering a therapeutically effective amount of a remedy selected from the group consisting of the agent according to any one of Claims 1 to 6, the vector according to either Claim 8 or 9, the anti-prion chimera antibody according to either Claim 10 or 11, and the preparation according to any one of Claims 19 to 23.

25. A method of treating a prion disease, the method comprising sustainedly releasing an anti-prion antibody.

26. The method according to Claim 25, wherein it comprises administering the preparation according to any one of Claims 19 to 23 and/or the vector according to either Claim 8 or 9.

27. The method according to Claim 25, wherein it comprises introducing an anti-prion antibody gene into target cells.

28. Use of mesenchymal cells in producing an agent for delivering a substance to a lesion site of a prion disease.

29. An agent for delivering a substance to a lesion site of a prion disease, the agent comprising mesenchymal cells.

30. A method for delivering a substance to a lesion site of a prion disease using mesenchymal cells.
